# EUROPEAN PATENT APPLICATION

(11) **EP 4 585 215 A1**
(43) Date of publication of application: **16.07.2025**
(21) Application number: 23862377.1
(22) Date of filing: 05.09.2023
(51) Int. Cl.: A61K 31/538, A61K 31/4704, A61K 31/46, A61K 31/167, A61P 11/00, A61P 11/08

(54) **PHARMACEUTICAL COMPOSITION FOR INHALATION FOR PREVENTING OR TREATING RESPIRATORY DISEASE**

(30) Priority: 05.09.2022 CN 202211078968
(71) Applicant: Lexenpharm (Suzhou) Limited, Suzhou, Jiangsu 215123 (CN)
(72) Inventor: LEE, Junning, Suzhou, Jiangsu 215123 (CN); LIU, Yang, Suzhou, Jiangsu 215123 (CN)
(74) Representative: Schulz Junghans Patentanwälte PartGmbB
(86) International application number: PCT/CN2023/116985
(87) International publication number: WO 2024/051683

(57) **Abstract**

Disclosed is a pharmaceutical composition for inhalation, comprising long-acting β₂ agonist and long-acting muscarinic antagonist. The long-acting β₂ agonist comprises olodaterol; or long-acting β₂ agonist comprises arformoterol, and the pH of the composition is 3.5-5.5; or long-acting β₂ agonist comprises indacaterol, and the pH of the composition is 2.5-4.5; or the long-acting muscarinic antagonist comprises revefenacin, and the pH of the composition is 3.5-5.5; or the long-acting muscarinic antagonist comprises glycopyrronium bromide, and the pH of the composition is 2.5-4.5; or the long-acting muscarinic antagonist comprises tiotropium bromide, and the pH of the composition is 2.0-4.0. Disclosed is a use of the pharmaceutical composition for inhalation for the preventing or treating of respiratory diseases such as asthma, chronic obstructive pulmonary disease, and the like.

## Description

### TECHNICAL FIELD

The present invention pertains to the field of drugs for preventing or treating diseases. More specifically, it relates to a pharmaceutical composition for inhalation for preventing or treating respiratory diseases such as asthma and chronic obstructive pulmonary disease.

### BACKGROUND

Chronic obstructive pulmonary disease (COPD) is a serious respiratory condition that is increasing in prevalence worldwide. The estimated prevalence in China is more than 100 million and it is currently the fourth leading cause of death in the U.K. and the U.S. It was the third most affected disease in the world by 2020.

COPD is a preventable and treatable disease, characterized by an incomplete reversible airflow limitation. Airflow obstruction is usually progressive and is associated with an abnormal inflammatory response of the lungs to toxic particles or gases, mainly caused by smoking. Although COPD affects the lungs, it also has significant systemic effects. COPD is associated with hypersecretion of mucus, emphysema, and bronchitis.

The main goals of COPD treatment include quitting smoking, relieving symptoms, improving physiology, and limiting complications, such as the abnormal ventilation and the onset of disease. However, the whole approach to treatment of COPD involves maintaining a combination of health care, such as quitting smoking, avoidance of indoor and outdoor pollutants and allergens, and avoidance of occupational exposure to allergens, progressive use of medications and complementary treatments as the disease progresses.

Currently, therapies to treat or prevent COPD and asthma include the use of long-acting bronchodilators or one or more inhaled corticosteroids (ICS).

Currently, therapies to treat or prevent COPD and asthma include the use of one or more combinations of long-acting bronchodilators and inhaled corticosteroids.

Inhaled bronchodilators are the foundation of COPD therapy because of their ability to relieve symptoms, reduce disease onset and improve quality of life. These drugs also improve airflow restriction and hyperinflation, which reduces respiratory activity and improves exercise tolerance. Moreover, bronchodilators can reduce respiratory muscle fatigue and improve mucociliary clearance.

More specifically, bronchodilators include β₂ agonist and anticholinergic. And the β₂ agonist may be short-acting for immediate relief of asthma symptoms, or long-acting for long-term prevention of asthma symptoms.

Long-acting β₂ agonist (LABA) or ultra-long-acting β₂ agonist (ULABA) has been shown to improve lung function, reduce symptoms, and protect against exercise-induced dyspnea in patients with asthma and COPD. LABA causes bronchiectasis by causing prolonged relaxation of the smooth muscle of the airway. In addition to prolonging bronchiectasis, LABA also play other roles, such as inhibiting airway smooth muscle cell proliferation and inflammatory mediators release, as well as non-smooth muscle effects, such as stimulating mucosal ciliary transport function, respiratory mucosa cell protection, and weakening neutrophil recruitment and activation.

In addition, the use of LABA reduces the frequency of medication taking. Commercially available twice-daily LABA includes salmeterol, formoterol, arformoterol, and the commercially available once-daily ULABA includes indacaterol, vilanterol, carmoterol and olodaterol.

Anticholinergic is also used as bronchodilator and is potential substitute for β₂ agonist, especially LABA. However, anticholinergic may also be taken with LABA to treat asthma. Anticholinergic work by competing with acetylcholine for receptor sites at the vagus nerve or neuromuscular junction. This prevents the transmission of reflexes induced by asthma irritants.

The use of anticholinergic drug offers advantages in older patients, as the responsiveness of β₂ agonist declines with age. And it would be advantageous that using anticholinergic in patients who do not tolerate the use of β₂ agonist.

Although β₂ agonist and anticholinergic provide symptomatic relief from bronchoconstriction, the COPD, as another element of inflammation, needs to be treated separately with steroids. Most inhaled corticosteroids require multiple dosing.

Corticosteroid has been shown to inhibit inflammatory cells and mediators involved in the pathology of respiratory conditions such as COPD. Treatment with corticosteroid/glucocorticoid is considered to be one of the most promising and effective therapies currently available for COPD.

However, corticosteroid use has been limited due to possible side effects associated with their use, including inhibition of the hypothalamic-pituitary-adrenal (HPA) axis, adverse effects on bone growth in children and bone density in the elderly, ophthalmic complications (cataract formation and glaucoma), and skin atrophy.

Treatment of acute exacerbation of COPD is still limited, including bronchodilators, inhaled corticosteroids and antibiotics. New and effective anti-inflammatory drugs are needed. According to the results of a preliminary study published in Respiratory Research, inhaling high molecular weight hyaluronic acid can shorten the duration of respiratory failure and non-invasive ventilation in patients with acute exacerbations of COPD. High molecular hyaluronic acid is a naturally occurring sugar, abundant in the cell matrix, included in the lungs. It has multiple properties that make it an attractive candidate as a therapeutic agent for acutely aggravated COPD.

There are currently a number of commercially available a pharmaceutical composition for inhalation comprising a combination of LABA and inhaled corticosteroid. Examples of the combination used to treat asthma and chronic obstructive pulmonary disease are salmeterol/ fluticasone propionate (Advair^{®} diskus and Advair^{®} HFA) and formoterol fumarate dehydrate/budesonide.

Therefore, in patients affected by respiratory conditions such as COPD, combined therapy with bronchodilators and ICS improves lung efficiency, reduces inflammatory response, and provides symptom relief compared to high doses of ICS itself. The selection of specific bronchodilators and ICS plays a very important role in the formulation of fixed-dose combination therapy.

Moreover, combination therapy reduces costs and provides control over respiratory conditions. Minimizing dose frequency is a major step in simplifying COPD treatment in order to improve patient adherence to therapy.

US 2009/0088408 A1 discloses a pharmaceutical composition of anticholinergic drugs, corticosteroids and P-mimics and use thereof in treating respiratory diseases. An example of the application is an inhalable powder or suspended aerosol composition comprising tiotropium bromide or ipratropium bromide.

US 2005/0042174 A1 discloses the combination administration of a β₂ agonist, an anticholinergic drug, and an anti-inflammatory steroid.

WO 2006/105401 A1 discloses anticholinergic agents in combination with corticosteroid and long-acting β₂ agonist, for use in preventing or treating respiratory, inflammatory, or obstructive airway diseases by simultaneously or sequentially administration.

US 2008/0279948 A1 discloses a drug comprising a β₂ agonist, glycopyrronium bromide, and mometasone furoate. An example of the application includes the β₂ agonist indacaterol maleate.

US 2008/0286363 A1 discloses a drug comprising β₂ agonist (such as indataterol maleate), glycopyrronium bromide, and corticosteroid. An example of the application includes corticosteroid: (6*S*,9*R*,10*S*,11*S*,13*S*,16*R*,17*R*)-9-chloro-6-fluoro-11-hydroxy-17-methoxycarbonyl-10,13,16-trimethyl-3-oxo-6, 7,8,9,10,11,12,13,14,15,16,17-dodecahydrogen-3*H*-cyclopenta[*a*]phenanthrene-17-yl 3-methylthiophene-2-carboxylate.

US 2010/0166671 A1 discloses a drug comprising antitoxycline, β₂ agonists, and corticosteroid. An example of the application includes glycopyrronium bromide, formoterol fumarate, and mometasone furoate.

US 7,439,393B2 discloses certain phenethanolamine derivatives for treating respiratory diseases, and their application in combination therapy with other therapeutic agents.

US 2008/0041369 A1 discloses a propellantless aerosol formulation comprising, in particular, olodaterol, corticosteroid (such as budesonide, beclometasone or fluticasone) and anticholinergic (such as tiotropium bromide, oxytropium bromide or ipratropium).

US 2005/0239778 A1 discloses drug combinations that specifically comprise olodaterol and at least one other active substance, such as a steroid.

US 2008/0317862 A1 discloses a drug comprising antidoxine and corticosteroid for treating inflammatory or obstructive airway diseases. In particular, the application disclosed an aerosol composition comprising glycopyrronium bromide and mometasone furoate.

US 2006/0069073 A1 discloses a combination of glycopyrronium bromide with one or more steroids as a second active substance.

CN 112804997A discloses an inhaled solution of a combination of indacaterol maleate and glycopyrronium bromide.

TW 202012369 A1 and CN 111936124A discloses an aerosol preparation without a booster, including, in particular, a pharmaceutical acceptable salt of glycopyrronium bromide, a pharmaceutical acceptable salt of indacaterol and water. It was also disclosed that the combination is particularly suitable for the application of aerosol formation of active drugs with the help of nebulizers for treating asthma and COPD.

CN 109715160 A discloses an aerosol comprising indacaterol or a derivative thereof, further comprising at least one pharmaceutically acceptable salt of glycopyrronium bromide; and/or at least one corticosteroid selected from the group consisting of budesonide, mometasone, beclomethasone, fluticasone, and pharmaceutically acceptable salts thereof.

US 2021/0322311 A1 discloses a liquid preparation without a booster, comprising, in particular, tiotropium bromide and olodaterol and pharmaceutically acceptable salts thereof.

US 7,056,916 B2 discloses a inhaled pharmaceutical formulation comprising olodaterol, including inhaled powders, aerosols comprising a booster, or inhaled solution without a booster.

US 2020/0368214 A1 discloses a propellant-free inhalation spray comprising a pharmaceutical combination selected from vilanterol, or umeclidinium bromide, or both vilanterol and umeclidinium bromide

WO 2021/009573 A1 discloses a pressure vessel with an internal coating of a polymer or copolymer, which in particular comrpises the drug umeclidinium bromide or vilanterol or a combination of both.

WO 2020/100040 A1 discloses an inhaler containing a booster and, in particular, a combination and formulation of umeclidinium bromide and vilanterol.

CN 112752572 A discloses a liquid, propellant-free drug preparation and a method of administering the drug by atomizing the drug preparation with an inhaler. The liquid, propellantless pharmaceutical preparation includes, in particular, an active substance selected from umeclidinium bromide, vilanterol triphenylacetate and a combination thereof.

WO 2020/220855 A1 discloses a propellant-free nebulized inhalation formulation comprising a pharmaceutical combination of umeclidinium bromide and vilanterol, wherein the formulation exhibits good stability and the atomized droplets have a particle size suitable for pulmonary inhalation.

CN 113274596 A discloses a pressurized metered-dose canister for treating respiratory diseases, comprising a corticosteroid, a long-acting β₂-agonist, a long-acting muscarinic antagonist and a propellant selected from HFA152a or HFO. Specifically, the aerosol formulation includes formoterol, beclomethasone, and glycopyrronium bromide as active ingredients

CN 111150728A discloses a stable pressurized aerosol solution comprising glycopyrronium bromide and formoterol.

WO 2020/229966 A1 discloses a stable suspension aerosol comprising glycopyrronium bromide. The formulation composition of the aerosol further includes one or more β₂ agonist. An example of the application is a compound aerosol comprising glycopyrronium bromide and formoterol.

CN 112804991 A discloses a propellant-free liquid formulation and a method of use via inhaler nebulization. Specifically, the formulation is a combination aerosol of umeclidinium bromide and formoterol.

WO 2020/084549 A1 discloses a composition and the method for preparing of an aerosol inhalation comprising glycopyrronium bromide and formoterol for treating pneumonia and obstructive tracheal diseases.

US 2018/0104184 A1 discloses a composition and method for preparation of an aerosol inhalation containing tiotropium bromide and formoterol for treating pneumonia and obstructive tracheal diseases.

US 6,433,027 B1 discloses a novel pharmaceutical composition concerning an anticholinergic compound and its preparation process. An example of the application is an inhalation spray comprising tiotropium bromide and formoterol.

CN 111481550A discloses a pharmaceutical combination comprising tiotropium bromide and arformoterol. Examples of applications include aerosols and inhalers comprising these two active ingredients.

WO 2010/048384 A1 discloses a composition and method for preventing and/or treating airway and/or respiratory diseases. In particular, the application discloses pharmaceutical composition for inhalation comprising arformoterol ((R,R)-formoterol) and tiotropium bromide.

CN 107233311 A discloses a composition and method for preparation of an aerosol inhalation comprising arformoterol and glycopyrronium bromide.

US 2017/0027908 A1 or JP 2017-061456 A discloses a pharmaceutical composition for inhalation for preventing and/or treating respiratory, inflammatory or obstructive airway diseases. In particular, it includes glycopyrronium bromide, β₂ agonist, and corticosteroid.

### SUMMARY OF THE INVENTION

A pharmaceutical composition for inhalation comprising one or more bronchodilators, in particular comprising a long-acting β₂ agonist and a long-acting muscarinic antagonist, is provided. In some embodiments, the use of one or more bronchodilators, in particular long-acting β₂ agonists and long-acting muscarinic antagonists, in the preparation of a pharmaceutical composition for inhalation for preventing and/or treating respiratory diseases, is provided. In some embodiments, a method for preparing the pharmaceutical composition is further provided. In some embodiments, the use of the pharmaceutical composition in preventing and/or treating respiratory diseases is further provided. In some embodiments, the pharmaceutical composition is for used in preventing and/or treating respiratory diseases. In some embodiments, a method for preventing and/or treating respiratory diseases, comprising the administration of the pharmaceutical composition, is provided.

In some embodiments, the respiratory disease comprises respiratory, inflammatory, or obstructive airway diseases. In some embodiments, the respiratory disease comprises asthma. In some embodiments, the respiratory disease comprises chronic obstructive pulmonary disease.

In some embodiments, the long-acting β₂ agonist comprises or consists of olodaterol. In some embodiments, the long-acting β₂ agonist comprises or consists of arformoterol, and the pH of the composition is 3.0-5.5, such as 3.5-5.5, such as 4.0-5.5, and such as 4.5-5.5. In some embodiments, the long-acting β₂ agonist comprises or consists of indacaterol, and the pH of the composition is 2.5-4.5, such as 3.0-4.5, such as 2.5-4.0, and such as 3.0-4.0. In some embodiments, long-acting muscarinic antagonist comprises or consists of revefenacin, and the pH of the composition is 3.5-5.5, such as 4.0-5.5, and such as 4.5-5.5. In some embodiments, long-acting muscarinic antagonist comprises or consists of glycopyrronium bromide, and the pH of the composition is 2.5-4.5, such as 2.5-4.0, and such as 2.5-3.5. In some embodiments, long-acting muscarinic antagonist comprises or consists of tiotropium, and the pH of the composition is 2.0-4.0, such as 2.5-4.0, and such as 2.5-3.5.

In some embodiments, the long-acting β₂ agonist comprises or consists of at least one selected from the group consisting of indacaterol, formoterol, arformoterol, vilanterol, carmoterol, and olodaterol. In some embodiments, the long-acting muscarinic antagonist comprises or consists of at least one selected from the group consisting of glycopyrronium bromide, umeclidinium, tiotropium, aclidinium bromide, and revefenacin.

In some embodiments, the long-acting β₂ agonist comprises indacaterol, and the long-acting muscarinic antagonist comprises glycopyrronium bromide. In some embodiments, the long-acting β₂ agonist comprises indacaterol, and the long-acting muscarinic antagonist comprises umeclidinium bromide. In some embodiments, the long-acting β₂ agonist comprises indacaterol, and the long-acting muscarinic antagonist comprises tiotropium bromide. In some embodiments, the long-acting β₂ agonist comprises indacaterol, and the long-acting muscarinic antagonist comprises aclidinium bromide. In some embodiments, the long-acting β₂ agonist comprises indacaterol, and the long-acting muscarinic antagonist comprises revefenacin. In some embodiments, the long-acting β₂ agonist comprises formoterol, and the long-acting muscarinic antagonist comprises glycopyrronium bromide. In some embodiments, the long-acting β₂ agonist comprises formoterol, and the long-acting muscarinic antagonist comprises umeclidinium bromide. In some embodiments, the long-acting β₂ agonist comprises formoterol, and the long-acting muscarinic antagonist comprises tiotropium bromide. In some embodiments, the long-acting β₂ agonist comprises formoterol, and the long-acting muscarinic antagonist comprises aclidinium bromide. In some embodiments, the long-acting β₂ agonist comprises formoterol, and the long-acting muscarinic antagonist comprises revefenacin. In some embodiments, the long-acting β₂ agonist comprises arformoterol, and the long-acting muscarinic antagonist comprises glycopyrronium bromide. In some embodiments, the long-acting β₂ agonist comprises arformoterol, and the long-acting muscarinic antagonist comprises umeclidinium bromide. In some embodiments, the long-acting β₂ agonist comprises arformoterol, and the long-acting muscarinic antagonist comprises tiotropium bromide. In some embodiments, the long-acting β₂ agonist comprises arformoterol, and the long-acting muscarinic antagonist comprises aclidinium bromide. In some embodiments, the long-acting β₂ agonist comprises arformoterol, and the long-acting muscarinic antagonist comprises revefenacin. In some embodiments, the long-acting β₂ agonist comprises vilanterol, and the long-acting muscarinic antagonist comprises glycopyrronium bromide. In some embodiments, the long-acting β₂ agonist comprises vilanterol, and the long-acting muscarinic antagonist comprises umeclidinium bromide. In some embodiments, the long-acting β₂ agonist comprises vilanterol, and the long-acting muscarinic antagonist comprises tiotropium bromide. In some embodiments, the long-acting β₂ agonist comprises vilanterol, and the long-acting muscarinic antagonist comprises aclidinium bromide. In some embodiments, the long-acting β₂ agonist comprises vilanterol, and the long-acting muscarinic antagonist comprises revefenacin. In some embodiments, the long-acting β₂ agonist comprises carmoterol, and the long-acting muscarinic antagonist comprises glycopyrronium bromide. In some embodiments, the long-acting β₂ agonist comprises carmoterol, and the long-acting muscarinic antagonist comprises umeclidinium bromide. In some embodiments, the long-acting β₂ agonist comprises carmoterol, and the long-acting muscarinic antagonist comprises tiotropium bromide. In some embodiments, the long-acting β₂ agonist comprises carmoterol, and the long-acting muscarinic antagonist comprises aclidinium bromide. In some embodiments, the long-acting β₂ agonist comprises carmoterol, and the long-acting muscarinic antagonist comprises revefenacin. In some embodiments, the long-acting β₂ agonist comprises olodaterol, and the long-acting muscarinic antagonist comprises glycopyrronium bromide. In some embodiments, the long-acting β₂ agonist comprises olodaterol, and the long-acting muscarinic antagonist comprises umeclidinium bromide. In some embodiments, the long-acting β₂ agonist comprises olodaterol, and the long-acting muscarinic antagonist comprises tiotropium bromide. In some embodiments, the long-acting β₂ agonist comprises olodaterol, and the long-acting muscarinic antagonist comprises aclidinium bromide. In some embodiments, the long-acting β₂ agonist comprises olodaterol, and the long-acting muscarinic antagonist comprises revefenacin.

In some embodiments, in the pharmaceutical composition, the long-acting β₂ agonist has a concentration of 1-100 µg/mL, such as 5-100 µg/mL, such as 5, 5.5, 6, 6.5, 7, 7.5, 8, 8.5, 9, 9.5, 10, 10.5, 11, 11.5, 12, 12.5, 13, 14, 15, 16, 17, 18, 19, 20, 22, 24, 25, 26, 28, 30, 35, 40, 45, 50, 55, 60, 65, 70, 75, 80, 85, 90, 95 or 100 µg/mL or the range between any two values. In some embodiments, in the pharmaceutical composition, the long-acting muscarinic antagonist has a concentration of 1-100 µg/mL, such as 5-100 µg/mL, such as 5, 5.5, 6, 6.5, 7, 7.5, 8, 8.5, 9, 9.5, 10, 10.5, 11, 11.5, 12, 12.5, 13, 14, 15, 16, 17, 18, 19, 20, 22, 24, 25, 26, 28, 30, 35, 40, 45, 50, 55, 60, 65, 70, 75, 80, 85, 90, 95 or 100 µg/mL or the range between any two values. In some embodiments, the weight ratio of the long-acting β₂ agonist to the long-acting muscarinic antagonist is 100:1-1:100, such as 1: 80-80 : 1, such as 1: 60-60 : 1, such as 1: 50-50 : 1, such as 1: 40-40 : 1, such as 1: 30-30 : 1, such as 1: 20-20: 1, such as 1: 15-15 : 1. In some embodiments, the weight ratio of the long-acting β₂ agonist to the long-acting muscarinic antagonist is 100:1-1:1, such as 1: 80-1 : 1, such as 1: 60-1 : 1, such as 1: 50-1 : 1, such as 1: 40-1 : 1, such as 1: 30-1: 1, such as 1: 20-1 : 1, such as 1: 15-1 : 1. In some embodiments, the weight ratio of the long-acting β₂ agonist to the long-acting muscarinic antagonist is 1:1-1:100, such as 1: 1-80 : 1, such as 1: 1-60 : 1, such as 1: 1-50 : 1, such as 1: 1-40 : 1, such as 1: 1-30 : 1, such as 1: 1-20 : 1, such as 1: 1-15 : 1.

In some embodiments, the composition further comprises hyaluronic acid. In some embodiments, the hyaluronic acid is a high molecular weight hyaluronic acid (HMW-HA). In some embodiments, the percentage of the hyaluronic acid by weight is less than 0.500%. In some embodiments, the percentage by weight of the hyaluronic acid is 0.001%-0.500%, such as 0.010%-0.400 %, such as 0.100%-0.300 %, such as 0.300%. In some embodiments, molecular weight of the high molecular weight hyaluronic acid ranges from 1,000,000 to 10,000,000 Dalton.

In some embodiments, the pharmaceutical composition further comprises sodium chloride, ethylenediamine tetraacetic acid (EDTA), and/or cyclodextrin. In some embodiments, the pharmaceutical composition further comprises sodium chloride. In some embodiments, the pharmaceutical composition further comprises ethylenediamine tetraacetic acid (EDTA), or cyclodextrin. In some embodiments, the pharmaceutical composition further comprises EDTA. In some embodiments, the pharmaceutical composition further comprises cyclodextrin. In some embodiments, the pharmaceutical composition further comprises EDTA, and/or cyclodextrin. In some embodiments, the pharmaceutical composition further comprises Tween, such as Tween 80. In some embodiments, the pharmaceutical composition further comprises ethyl oleate. In some embodiments, the pharmaceutical composition further comprises glucocorticoid. In some embodiments, the cyclodextrin comprises α-cyclodextrin, β-cyclodextrin, or γ-cyclodextrin. In some embodiments, the cyclodextrin is select from the group consisting of α-cyclodextrin, β-cyclodextrin, and γ-cyclodextrin. In some embodiments, the cyclodextrin comprises or consists of α-cyclodextrin. In some embodiments, the cyclodextrin comprises or consists of β-cyclodextrin. In some embodiments, the cyclodextrin comprises or consists of γ-cyclodextrin. In some embodiments, the cyclodextrin comprises hydroxypropyl-β-cyclodextrin or sulfobutyl-β-cyclodextrin. In some embodiments, the cyclodextrin is selected from the group consisting of hydroxypropyl-β-cyclodextrin and sulfobutyl-β-cyclodextrin. In some embodiments, the cyclodextrin comprises or consists of hydroxypropyl-β-cyclodextrin. In some embodiments, the cyclodextrin comprises or consists of sulfobutyl-β-cyclodextrin.

In some embodiments, the sodium chloride has a weight percentage of less than 1.00%. In some embodiments, the sodium chloride has a weight percentage of 0.01%-1.00%, such as 0.10%-1.00 %, such as 0.50%-1.00 %, such as 0.85%. In some embodiments, the EDTA has a weight percentage of less than 0.100%. In some embodiments, the EDTA has a weight percentage of 0.001%-0.100%, such as 0.010%-0.050 %, such as 0.020%-0.040 %, such as 0.030%. In some embodiments, the cyclodextrin has a weight percentage of less than 10%, such as less than 5.0%, such as less than 2.5%, such as less than 1.50%. In some embodiments, the percentage by weight of the cyclodextrin is 0.01%-10 %, such as 0.01%-5.0 %, such as 0.01%-2.5 %, such as 0.01%-1.50 %, such as 0.01%-1.00 %, such as 0.10%-1.00 %, such as 0.20%-0.80 %, such as 0.25%-0.75 %, such as 0.30%-0.70 %, such as 0.40%-0.60 %, such as 0.50%. In some embodiments, the weight percentage of Tween is less than 0.100%. In some embodiments, the weight percentage of the Tween is 0.001%-0.100 %, such as 0.010%-0.050 %, such as 0.020%-0.040 %, such as 0.030%.

In some embodiments, the pharmaceutical composition further comprises a buffer, such as a citric acid/citrate buffer, and for example, a citric acid/sodium citrate buffer. In some embodiments, the pharmaceutical composition further comprises a pH regulator, such as hydrochloric acid, and for example, a dilute hydrochloric acid.

In some embodiments, the long-acting β₂ agonist comprises olodaterol, and the long-acting muscarinic antagonist comprises revefenacin. In some embodiments, the long-acting β₂ agonist comprises olodaterol, the long-acting muscarinic antagonist comprises revefenacin, and the pH of the composition is 4.0-5.5. In some embodiments, the long-acting β₂ agonist comprises olodaterol, the long-acting muscarinic antagonist comprises revefenacin, and the composition further comprises EDTA.

In some embodiments, the long-acting β₂ agonist comprises arformoterol, the long-acting muscarinic antagonist comprises revefenacin, and the pH of the composition is 4.0-5.5, such as 4.5-5.5, or such as 4.0-5.0. In some embodiments, the long-acting β₂ agonist comprises arformoterol, the long-acting muscarinic antagonist comprises revefenacin, and the composition further comprises EDTA. In some embodiments, the long-acting β₂ agonist comprises arformoterol, the long-acting muscarinic antagonist comprises revefenacin, and the composition further comprises buffer, such as citric acid/citrate buffer, and such as citric acid/sodium citrate buffer. In some embodiments, the long-acting β₂ agonist comprises arformoterol, the long-acting muscarinic antagonist comprises revefenacin, and the composition further comprises buffer, such as citric acid/citrate buffer, and such as citric acid/sodium citrate buffer, and the concentration of buffer solution is more than 0.1 mM, such as 0.1 mM to 20 mM, such as 0.1mM-10 mM, such as 1 mM-10 mM. In some embodiments, the long-acting β₂ agonist comprises arformoterol, the long-acting muscarinic antagonist comprises revefenacin, and the composition further comprises sodium chloride, such as sodium chloride having a weight percentage of less than 1.00%, such as 0.01% to 1.00%.

In some embodiments, the long-acting β₂ agonist comprises indacaterol, the long-acting muscarinic antagonist comprises glycopyrronium bromide, and the pH of the composition is 2.5 to 4.0, such as 3.0-4.0, such as 2.5-3.5, such as 3.0-3.5, such as 3.5. In some embodiments, the long-acting β₂ agonist comprises indacaterol, the long-acting muscarinic antagonist comprises glycopyrronium bromide, and the composition further comprises EDTA and/or cyclodextrin, such as comprises cyclodextrin, such as comprises EDTA and cyclodextrin. In some embodiements, the long-acting β₂ agonist comprises indacaterol, the long-acting muscarinic antagonist comprises glycopyrronium bromide, and the composition further comprises sodium hyaluronate. In some embodiements, the long-acting β₂ agonist comprises indacaterol, the long-acting muscarinic antagonist comprises glycopyrronium bromide, and the composition further comprises sodium hyaluronate and at least one of the group consisting of EDTA and cyclodextrin. In some embodiements, the long-acting β₂ agonist comprises indacaterol, the long-acting muscarinic antagonist comprises glycopyrronium bromide, and the composition further comprises sodium hyaluronate and cyclodextrin.

In some embodiments, the long-acting β₂ agonist comprises indacaterol, the long-acting muscarinic antagonist comprises revefenacin, and the pH of the composition is 3.0-4.5, such as 3.5-4.5, such as 3.5-4.0, such as 4.0-4.5, such as 4.0. In some embodiments, the long-acting β₂ agonist comprises indacaterol, the long-acting muscarinic antagonist comprises revefenacin, and the composition further comprises EDTA and/or cyclodextrin, such as comprises cyclodextrin, such as comprises EDTA and cyclodextrin.

In some embodiments, the long-acting β₂ agonist comprises indacaterol, the long-acting muscarinic antagonist comprises tiotropium bromide, and the pH of the composition is 2.0-4.0, such as 2.5-4.0, such as 2.5-3.5, such as 2.5-3.0, such as 2.5. In some embodiments, the long-acting β₂ agonist comprises indacaterol, the long-acting muscarinic antagonist comprises tiotropium bromide, and the composition further comprises cyclodextrin.

In some embodiments, the composition is a pharmaceutical composition for aerosol inhalation. In some embodiments, the composition is an atomized pharmaceutical composition for inhalation administered by a vibrating screen, ultrasonic spray, or air compressor.

In some embodiments, the composition is a soft mist, an aerosol or a nebulizer.

In some embodiments, the composition is a metered-dose inhaler.

In some embodiments, the pharmaceutical composition further comprises one or more of an excipient, an ejector, a co-solvent, a filler, a nonvolatile component, a buffer, a pH regulator, a surfactant, a preservative, a complexer, an antioxidant, or a combination thereof.

In some embodiments, the composition is a soft mist, the method for preparing the soft mist comprises mixing a bronchodilator and a high molecular weight hyaluronic acid, in particular mixing a long-acting β₂ agonist, a long-acting muscarinic antagonist, and a high molecular weight hyaluronic acid, in a solvent.

In some embodiments, the pharmaceutical composition is an aerosol, the method for preparing the aerosol comprising mixing a bronchodilator, a high molecular weight hyaluronic acid, and a projectile in a solvent, in particular a mixture of a long-acting β₂ agonist, a long-acting muscarinic antagonist, a high molecular weight hyaluronic acid, and a projectile. In some embodiments, the ejector is a hydrofluoroalkane (HFA). In some embodiments, the HFA is 1,1,1,2-tetrafluoroethane and/or 1,1,1,2,3,3,3-heptafluoropropane. In some embodiments, the HFA is 1, 1, 1,2-tetrafluoroethane. In some embodiments, the HFA is 1,1,1,2,3,3,3-heptafluoropropane. In some embodiments, the HFA is 1,1,1,2-tetrafluoroethane and 1,1,1,2,3,3,3-heptafluoropropane.

In some embodiments, the active substance of the pharmaceutical composition is present in a fixed or free combination for simultaneous, sequential, or separate administration in a form of the drug suitable for inhalation applications, together with the excipient.

In some embodiment of the present application, the mention of the long-acting β₂ agonist, long-acting muscarinic antagonist or other components, such as indacaterol, formoterol, arformoterol, vilanterol, cammoterol, and olodaterol, glycopyrronium bromide, umeclidinium bromide, tiotropium bromide, aclidinium bromide, and revefenacin, and hyaluronic acid such as high molecular weight hyaluronic acid, EDTA, ethyl oleate, *etc.*, all includes the mention of the pharmaceutically acceptable salts thereof, unless otherwise specified. For example, the mention in the embodiments of the present application that the pharmaceutical compositions of the present application comprise long-acting β₂ agonist and long-acting muscarinic antagonist, shall be deemed to refer to that, it is mentioned in these embodiments of the present application that the pharmaceutical compositions of the present application comprises long-acting β₂ agonist or pharmaceutically acceptable salts thereof, and long-acting muscarinic antagonist or pharmaceutically acceptable salts thereof; the mention in the enbodiments of the present application that the pharmaceutical compositions comprise EDTA, shall be deemed to refer to that, it is mentioned in these embodiments of the present application that the pharmaceutical composition of the present application comprises EDTA and pharmaceutically acceptable salts thereof; and so on.

### BRIEF DESCRIPTION OF THE DRAWINGS

FIG. 1 illustrates the change in the content of revefenacin during the stability test of the arformoterol tartrate-revefenacin compositions with different buffer concentrations.
FIG. 2 illustrates the change in the content of arformoterol during the stability test of the arformoterol tartrate-revefenacin compositions with different buffer concentrations.
FIG. 3 illustrates the change in pH value during the stability test of the arformoterol tartrate-revefenacin compositions with different buffer concentrations.
FIG. 4 illustrates the change in the content of revefenacin during the stability test of the arformoterol tartrate-revefenacin compositions with different excipients.
FIG. 5 illustrates the change in the content of arformoterol during the stability test of the arformoterol tartrate-revefenacin compositions with different excipients.
FIG. 6 illustrates the change in the content of indacaterol during the stability test of the indacaterol tartrate-glycopyrronium bromide compositions with different pH value.
FIG. 7 illustrates the change in the content of glycopyrronium bromide during the stability test of the indacaterol tartrate-glycopyrronium bromide compositions with different excipients.
FIG. 8 illustrates the change in the total impurities during the stability test of the indacaterol tartrate-glycopyrronium bromide compositions with different excipients.
FIG. 9 illustrates the change in the contents of indacaterol and glycopyrronium bromide during the stability test of the indacaterol tartrate-glycopyrronium bromide compositions having a pH value of 4 with different excipients.
FIG. 10 illustrates the change in the total impurities during the stability test of the indacaterol tartrate-glycopyrronium bromide compositions having a pH value of 4 with different excipients.
FIG. 11 illustrates the variation in the contents of indacaterol and glycopyrronium bromide during the stability test of the indacaterol tartrate-glycopyrronium bromide compositions having a pH value of 3 with different excipients.
FIG. 12 illustrates the change in the total impurities during the stability test of the indacaterol tartrate-glycopyrronium bromide compositions having a pH value of 3 with different excipients.
FIG. 13 illustrates the change in the contents of indacaterol and glycopyrronium bromide during the stability test of the indacaterol tartrate-glycopyrronium bromide-sodium hyaluronate compositions having a pH value of 4 with different excipients.
FIG. 14 illustrates the change in the total impurities during the stability test of the indacaterol tartrate-glycopyrronium bromide-sodium hyaluronate compositions having a pH value of 4 with different excipients.
FIG. 15 illustrates the change in the contents of indacaterol and glycopyrronium bromide during the stability test of the indacaterol tartrate-glycopyrronium bromide-sodium hyaluronate compositions having a pH value of 3 with different excipients.
FIG. 16 illustrates the change in the total impurities during the stability test of the indacaterol tartrate-glycopyrronium bromide-sodium hyaluronate compositions having a pH value of 4 with different excipients.
FIG. 17 illustrates the change in the content of indacaterol during the stability test of the indacaterol tartrate-revefenacin compositions with different pH values.
FIG. 18 illustrates the change in the content of indacaterol during the stability test of the indacaterol tartrate-revefenacin compositions with different pH values.
FIG. 19 illustrates the change in total impurities during the stability test of the indacaterol tartrate-revefenacin compositions with different pH values.
FIG. 20 illustrates the change in the total impurities during the stability test of the indacaterol tartrate-revefenacin compositions having a pH value of 4 with different excipients.
FIG. 21 illustrates the change in the total impurities of indacaterol during the stability test of the indacaterol tartrate-tiotropium bromide compositions with different pH values.
FIG. 22 illustrates the change in the total impurities of tiotropium bromide during the stability test of the indacaterol tartrate-tiotropium bromide compositions with different pH values.
FIG. 23 illustrates the change in the total impurities during the stability test of the indacaterol tartrate-tiotropium bromide compositions having a pH value of 3 with different excipients.
FIG. 24 illustrates the change in the total impurities during the stability test of the indacaterol tartrate-tiotropium bromide compositions having a pH value of 3, 4, or 5 with or without sodium hyaluronate.

### DETAILED DESCRIPTION OF THE EMBODIMENTS

To further elaborate the technical methods and effectiveness of the present application to achieve the intended purpose, in combination with the attached drawings and better embodiments, the specific embodiments, structure, characteristics and efficacy under the present application are described in detail as follows:
As used in the present application, the term "and/or" refers to and cover any and all possible combinations of one or more associated listed items. When used in a list of two or more items, the term "and/or" indicate that any one of the listed items can be used alone, or any combination of two or more of the listed items can be used. For example, if a composition (or combination, construct, proposition, etc.) is described as comprising (or including) components (or conditions) A, B, C and/or D, then the composition (or combination, construct, proposition, *etc.*) may contain A alone, B alone, C alone, D alone, and a combination of A and B, a combination of A and C, a combination of A and D, a combination of B and C, a combination of B and D, a combination of C and D, a combination of A, B and C, a combination of A, B and D, a combination of A, C and D, a combination of B, C and D, a combination of A, B, C and D.

As used in the present application, including as used in embodiments and unless expressly specified otherwise, all numbers can be thought of as beginning with the words "essentially", "approximately" or "about", even if the term does not appear explicitly. When describing the magnitude and/or position to indicate that the values and/or positions described are within a reasonably expected range of values and/or positions, the phrase "approximately" or "about" may be used. For example, the value can be 0.1% of the value (or range of values), ±1% of the value (or range of values), ±2% of the value (or range of values), ±5% of the value (or range of values), ±10% of the value (or range of values), ±15% of the value (or range of values), ±20% of the value (or range of values). Any numerical range referred to in the present application is intended to include all subranges or intermediate values contained therein.

Disclosure of values and ranges of values for specific parameters does not exclude other values and ranges useful to the application. It can be expected that two or more concrete example values for a given parameter can determine the end points of the range of values that the parameter can achieve. For example, if the parameter *X* is exemplified in the present application as having a value *A* and is also exemplified as having a value *Z*, then the parameter *X* can be expected to have a numerical range from about *A* to about *Z*. Similarly, two or more value ranges of the exposed parameter (whether these ranges are nested, overlapping, or distinct) can be expected to include all possible combinations of value ranges that can be required using endpoints of the exposed range. For example, if the parameter *X* is exemplified in the present application as having values in the range 1-10, it also describes the subrange of the parameter *X*, including, for example only, 1-9, 1-8, 1-7, 2-9, 2-8, 2-7, 3-9, 3-8, 3-7, 2-8, 3-7, 4-6 or 7-10, 8-10 or 9-10. The range includes its endpoints and the values within them, for example, the range 0 to 5 includes 0, >0, 1, 2, 3, 4, <5 and 5.

### Example 1 Stability test of the olodaterol hydrochloride-revefenacin compositions at different pH and with the presence of EDTA

Buffers of pH 4.0, 4.5, 5.0, 5.5 (two for those at pH 5.0 and one for of the rest) were prepared with 5 mM (mmol/L) citric acid solution and sodium citrate solution, respectively. The prescribed amounts of sodium chloride were weighed and dissolved with 200 mL pH buffers, added with the prescribed amounts of olodaterol hydrochloride and revefenacin precisely, mixed well, and volumed with pH buffers to 250 mL. Then, EDTA was added to one of the pH 5.0 solution and stired until completely dissolved. The drug solutions were filled into low-density polyethylene plastic bottles at 2 mL per bottle. The experimental formulation is shown in Table 1.

**Table 1 Formula of the olodaterol hydrochloride-revefenacin compositions**

| components | Sample 1 | Sample 2 | Sample 3 | Sample 4 | Sample 5 |
|---|---|---|---|---|---|
| olodaterol hydrochloride (mg) | 17.1 | 17.1 | 17.1 | 17.1 | 17.1 |
| revefenacin (mg) | 21.25 | 21.25 | 21.25 | 21.25 | 21.25 |
| sodium chloride (g) | 2.1875 | 2.1875 | 2.1875 | 2.1875 | 2.1875 |
| citric acid/sodium citrate buffer | adjust to pH **4.0** | adjust to pH **4.5** | adjust to pH **5.0** | adjust to pH **5.5** | adjust to pH **5.0** |
| EDTA (mg) | **0** | **0** | **0** | **0** | **150.47** |
| water for injection (mL) | *q.s.* to 250 mL | | | | |

Stability tests were performed to determine the contents of olodaterol and revefenacin in the samples at initial and after 2 weeks at 60°C. The results are shown in Table 2.

**Table 2 Stability test results of the olodaterol hydrochloride-revefenacin compositions**

| | initial | | 2 weeks at 60°C | |
|---|---|---|---|---|
| | olodaterol content | revefenacin content | olodaterol content | revefenacin content |
| Sample 1 | 100.4% | 99.9% | 99.0% | 99.5% |
| Sample 2 | 99.0% | 99.3% | 97.7% | 99.7% |
| Sample 3 | 99.6% | 97.7% | 97.7% | 97.7% |
| Sample 4 | 98.4% | 98.8% | 96.5% | 98.7% |
| Sample 5 | 98.9% | 99.3% | 97.6% | 99.9% |

It can be seen that after all the samples were placed at 60°c for 2 weeks, the content of olodaterol changed within 2%, and the content of revefenacin almost did not change, and the stability was good.

### Example 2 Stability test of the arformoterol tartrate-revefenacin compositions at different pH

Three buffers at pH 4.0, 4.5 and 5.0 were prepared by mixing an appropriate amount of citric acid solution and sodium citrate solution respectively. 450 mL of each of the pH buffers were accurately added with the prescribed amounts of sodium chloride, arformoterol tartrate and revefenacin, and mixed, and volumed with pH buffers to 500 mL. The obtained drug solutions were filled into low-density polyethylene plastic bottles at 2 mL per bottle. The experimental formulation is shown in Table 3.

**Table 3 Formula of the arformoterol tartrate and revefenacin compositions**

| components | Sample 1 | Sample 2 | Sample 3 |
|---|---|---|---|
| arformoterol tartrate (mg) | 5.5 | 5.5 | 5.5 |
| revefenacin (mg) | 42.5 | 42.5 | 42.5 |
| sodium chloride (g) | 4.375 | 4.375 | 4.375 |
| citric acid/sodium citrate buffer | adjust to pH **4.0** | adjust to pH **4.5** | adjust to pH **5.0** |
| | *q.s.* to 500 mL | | |

Stability tests were performed to determine the contents of arformoterol and revefenacin in the samples at initial and after 4 weeks at 25±2°c. The results are shown in Table 4.

**Table 4 Stability test results of the arformoterol tartrate-revefenacin compositions**

| | initial | | 4 weeks at 25±2°C | |
|---|---|---|---|---|
| | arformoterol content | revefenacin content | arformoterol content | revefenacin content |
| Sample 1 | 101.1% | 98.4% | 98.6% | 98.0% |
| Sample 2 | 98.1% | 98.5% | 96.4% | 97.7% |
| Sample 3 | 99.0% | 97.7% | 97.2% | 97.4% |

It can be seen that after all the samples were placed at 25±2°C for 4 weeks, the content of arformoterol changed within 3%, and the content of revefenacin almost did not change, and the stability was well. The long-term storage condition of the commercially available arformoterol tartrate inhalation solution is 2-8 °C, the acceleration condition is 25±2°C, and the products can be stored at the acceleration condition for 3 months.

### Example 3 Stability test of the arformoterol tartrate-revefenacin-hyaluronic acid compositions at different pH and with different concentrations of EDTA

The prescribed amounts of sodium chloride were added to 450 mL of 5 mM citric acid/sodium citrate buffers (pH 4.5, 5.5, 5.0, 4.5, and 5.5, respectively). Stirred to dissolve, then added with the prescription amounts of precisely weighed arformoterol tartrate, revefenacin and hyaluronic acid, mixed well, and volumed with pH buffers to 500 mL. The obtained drug solutions were filled into low-density polyethylene plastic bottles at 2 mL per bottle. The experimental formulation is shown in Table 5.

**Table 5 Formula of the arformoterol tartrate-revefenacin-Hyaluronic acid compositions**

| components | Sample 1 | Sample 2 | Sample 3 | Sample 4 | Sample 5 |
|---|---|---|---|---|---|
| arformoterol tartrate (mg) | 5.5 | 5.5 | 5.5 | 5.5 | 5.5 |
| revefenacin (mg) | 42.5 | 42.5 | 42.5 | 42.5 | 42.5 |
| high molecular weight hyaluronic acid (g) | 1.5 | 1.5 | 1.5 | 1.5 | 1.5 |
| EDTA (mg) | **50** | **50** | **150** | **250** | **250** |
| sodium chloride(g) | 4.375 | 4.375 | 4.375 | 4.375 | 4.375 |
| citric acid/sodium citrate buffer | adjust to pH 4.5 | adjust to pH 5.5 | adjust to pH 5.0 | adjust to pH 4.5 | adjust to pH 5.5 |
| | *q.s.* to 500 mL | | | | |

Stability test was performed to determine the contents of arformoterol and revefenacin in the samples at initial and after 4 weeks at 25±2°C. The results are shown in Table 6.

**Table 6 Stability test results of the arformoterol tartrate-revefenacin-hyaluronic acid compositions**

| | initial | | 4 weeks at 25±2°C | |
|---|---|---|---|---|
| | arformoterol content | revefenacin content | arformoterol content | revefenacin content |
| Sample 1 | 102.6% | 98.2% | 100.1% | 98.3% |
| Sample 2 | 100.4% | 97.8% | 98.8% | 97.2% |
| Sample 3 | 105.7% | 99.4% | 100.9% | 98.1% |
| Sample 4 | 100.8% | 98.7% | 98.4% | 99.6% |
| Sample 5 | 105.1% | 100.8% | 103.5% | 99.2% |

It can be seen that after all the samples were placed at 25+2°C for 4 weeks, the content of arformoterol changed within 5%, and the content of revefenacin almost did not change.

### Sample 4 Stability test of arformoterol tartrate-revefenacin compositions in different buffer concentrations

1 L citric acid/sodium citrate buffers at pH 5 in different concentrations (where the pH is adjusted with dilute hydrochloric acid if no buffer is added) were prepared. Added with the prescribed amounts of sodium chloride, stirred to dissolve, and added with revefenacin and arformoterol tartrate, stirred until completely dissolved. The obtained drug solutions were filled into low-density polyethylene plastic bottles at 2 mL per bottle. The experimental formulation is shown in Table 7.

**Table 7 Formula of the arformoterol tartrate-revefenacin compositions**

| | Sample 1 | Sample 2 | Sample 3 | Sample 4 |
|---|---|---|---|---|
| revefenacin (µg/mL) | 25 | 25 | 25 | 25 |
| arformoterol tartrate (µg/mL) | 10 | 10 | 10 | 10 |
| sodium chloride (%, w/w) | 0.85 | 0.85 | 0.85 | 0.85 |
| citric acid/sodium citrate buffer (mm) | **0** | **0.1** | **1** | **10** |
| pH | 5 | 5 | 5 | 5 |

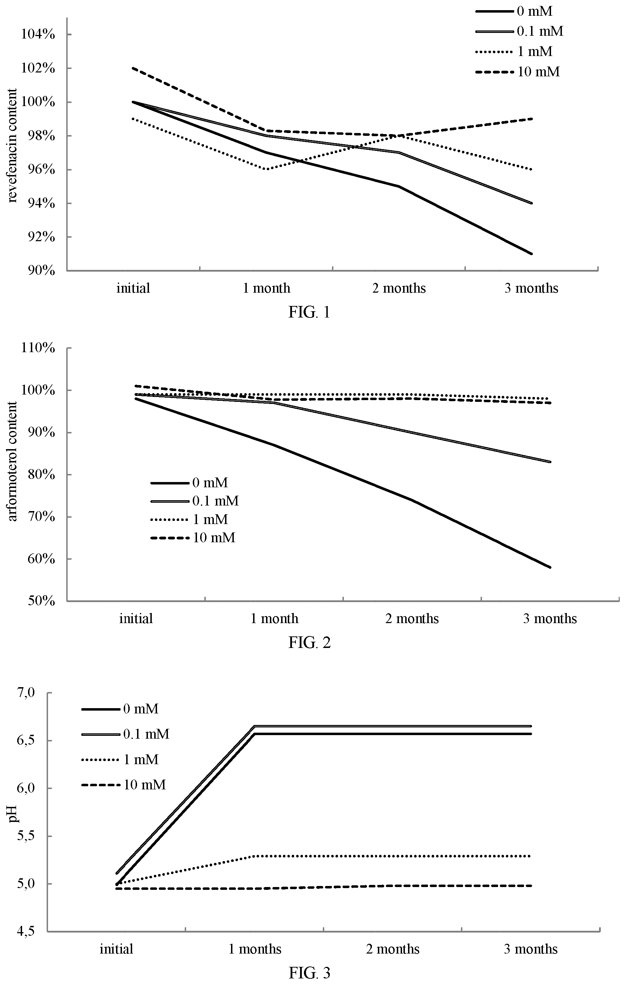

Stability test was carried out to determine the contents of arformoterol and revefenacin and the pH value of the samples at initial and after being placed at 25°C, 60% RH for 3 months. The results are shown in Table 8 and FIGs. 1 to 3. FIG. 1 shows the changes in the content of revefenacin during stability test of the arformoterol-revefenacin compositions in different buffer concentrations. FIG. 2 shows the changes in the content of arformoterol during stability test of the arformoterol-revefenacin compositions in different buffer concentrations. FIG. 3 shows the changes in the pH value during stability test of the arformoterol-revefenacin compositions in different buffer concentrations.

**Table 8 Stability test results of the arformoterol tartrate-revefenacin compositions**

| | revefenacin content | | | | arformoterol content | | | | pH value | | | |
|---|---|---|---|---|---|---|---|---|---|---|---|---|
| | 0 month | 1 month | 2 months | 3 months | 0 month | 1 month | 2 months | 3 months | 0 month | 1 month | 2 months | 3 months |
| Sample 1 | 100% | 97% | 95% | 91% | 98% | 87% | 74% | 58% | 5.0 | 6.6 | 6.6 | 6.6 |
| Sample 2 | 100% | 98% | 97% | 94% | 99% | 97% | 90% | 83% | 5.1 | 6.7 | 6.7 | 6.7 |
| Sample 3 | 99% | 96% | 98% | 96% | 99% | 99% | 99% | 98% | 5.0 | 5.3 | 5.3 | 5.3 |
| Sample 4 | 102% | 98% | 98% | 99% | 101% | 98% | 98% | 97% | 5.0 | 5.0 | 5.0 | 5.0 |

It can be seen that the addition of pH buffer is conducive to product stability. At the same time, as the pH buffer concentration increased, the stability of the active pharmaceutical ingredients rRevefenacin and arformoterol were significantly improved, and the pH was more stable during placement.

### Example 5 Stability test of the arformoterol tartrate-revefenacin compositions at different pH

1 mM citric acid/sodium citrate buffers at pH 4.5, 4.75, 5.25, 5.5 were prepared, added with the prescribed amounts of sodium chloride, stirred until completely dissolved. The prescribed amounts of revefenacin and arformoterol tartrate were added and stirred until dissolved. The obtained drug solutions were filled into low-density polyethylene plastic bottles at 2 mL per bottle, and then sealed with aluminum foil bags. The experimental formulation is shown in Table 9.

**Table 9 Formula of the arformoterol tartrate-revefenacin compositions**

| | Sample 1 | Sample 2 | Sample 3 | Sample 4 |
|---|---|---|---|---|
| revefenacin (µg/mL) | 12.5 | 12.5 | 12.5 | 12.5 |
| arformoterol (µg/mL) | 10 | 10 | 10 | 10 |
| sodium chloride (%, w/w) | 0.85 | 0.85 | 0.85 | 0.85 |
| pH | 4.5 | 4.75 | 5.25 | 5.5 |

Stability test was performed to determine the contents of arformoterol and revefenacin in the samples at initial and after 3 months at 40°C, 75% RH or 25°C, 60% RH. The results are shown in Table 10.

**Table 10 Stability test results of the arformoterol tartrate-revefenacin compositions**

| | 40°C, 75% RH | | | | 25°C, 60% RH | | | | 25°C, 60% RH | | | |
|---|---|---|---|---|---|---|---|---|---|---|---|---|
| | revefenacin content | | | | arformoterol content | | | | pH | | | |
| | 0 month | 1 month | 2 months | 3 months | 0 month | 1 month | 2 months | 3 months | 0 month | 1 month | 2 months | 3 months |
| Sample 1 | 100% | 98% | 96% | 97% | 101% | 98% | 97% | 94% | 4.5 | 4.4 | 4.5 | 4.5 |
| Sample 2 | 99% | 98% | 97% | 97% | 103% | 101% | 101% | 98% | 4.7 | 4.7 | 4.7 | 4.7 |
| Sample 3 | 100% | 98% | 98% | 98% | 102% | 100% | 101% | 99% | 5.3 | 5.2 | 5.2 | 5.2 |
| Sample 4 | 99% | 97% | 97% | 95% | 100% | 99% | 100% | 98% | 5.5 | 5.5 | 5.5 | 5.5 |

It can be seen that after being placed at 40°C, 75% RH for 3 months, the content of revefenacin in the compositions of arformoterol tartrate-revefenacin at a pH in a range between 4.5 and 5.5 reduces less than 5%, and the drug solutions are stable. The content of arformoterol in the composition of arformoterol tartrate-revefenacin at a pH in a range between 4.5 and 5.5 reduce less than 5% after being placed at 25°C, 60% RH for 2 months, and it is more sensitive to temperature compared to arformoterol solution, of which the commercially available products can be stored for 6 weeks at 25°C, 60% RH. It is further stated that in some embodiments, the composition is stable in the pH range of 4.5-5.5.

### Example 6 Stability test of the arformoterol tartrate-revefenacin compositions with excipients

In addition, the effects of sodium chloride, EDTA and Tween on the stability of arformoterol tartrate-revefenacin composition (10 mM citric acid-sodium citrate buffer at pH 5 as the solvent) were investigated. The experimental formula is shown in Table 11. The prepared drug solutions were filled into low-density polyethylene bottles, and then sealed with aluminum foil bags.

**Table 11 Formula of the arformoterol tartrate-revefenacin compositions**

| | Sample 1 | Sample 2 | Sample 3 | Sample 4 | Sample 5 |
|---|---|---|---|---|---|
| revefenacin (µg/mL) | 20 | 20 | 20 | 20 | 20 |
| arformoterol (µg/mL) | 7.5 | 7.5 | 7.5 | 7.5 | 7.5 |
| pH | 5 | 5 | 5 | 5 | 5 |
| sodium chloride (%, w/w) | none | 0.85 | 0.85 | 0.85 | 0.85 |
| EDTA (%, w/w) | none | none | none | 0.02 | 0.02 |
| tween (%, w/w) | none | none | 0.02 | none | 0.02 |

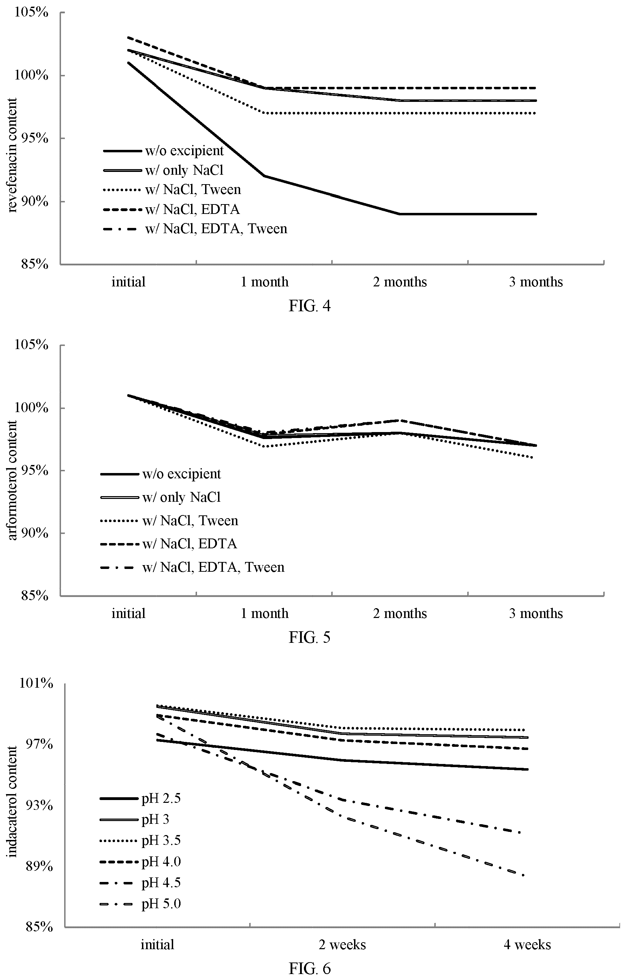

Stability test was performed to determine the contents of arformoterol and revefenacin in the samples and the pH value at initial state and after 3 months at 45°C, 75% RH. The results are shown in Table 12, FIG. 4 and FIG. 5. FIG. 4 shows the change of the content of revefenacin in the stability test of the arformoterol tartrate-revefenacin composition with different excipients. FIG. 5 shows the change of the content of arformoterol in the stability test of the arformoterol tartrate-revefenacin composition with different excipients.

**Table 12 Stability test results of the arformoterol tartrate-revefenacin compositions**

| | revefenacin content | | | | arformoterol content | | | |
|---|---|---|---|---|---|---|---|---|
| | 0 month | 1 month | 2 months | 3 months | 0 month | 1 month | 2 months | 3 months |
| Sample 1 | 101% | 92% | 89% | 89% | 101% | 98% | 98% | 97% |
| Sample 2 | 102% | 99% | 98% | 98% | 101% | 98% | 98% | 97% |
| Sample 3 | 102% | 97% | 97% | 97% | 101% | 97% | 98% | 96% |
| Sample 4 | 103% | 99% | 99% | 99% | 101% | 98% | 99% | 97% |
| Sample 5 | 102% | 99% | 98% | 98% | 101% | 98% | 99% | 97% |

It can be seen that the addition of excipient sodium chloride is significantly beneficial to the stability of revefenacin compared with the addition of no excipient or the addition of EDTA and Tween. The addition of sodium chloride, EDTA and Tween has no significant effect on the stability of arformoterol solution.

### Example 7 Stability test of the indacaterol maleate-glycopyrronium bromide compositions at different pH

The soft mist solutions of indatarol indacaterol maleate and glycopyrronium bromide at different pH were prepared as follows. Six 0.02mM citric acid solution each 500 mL were prepared. pH was adjusted to 2.5, 3.0, 3.5, 4.0, 4.5, and 5.0 with 1 M (mol/L) of sodium hydroxide solution. Indacaterol maleate and glycopyrronium bromide were added. In order to improve the dissolution of indacaterol, all the drug solutions were ultrasonicated in a water bath at 30°C for 20 min until no visible particles were found. The experimental formula is shown in Table 13.

**Table 13 Formula of the indacaterol maleate-glycopyrronium bromide compositions**

| | Sample 1 | Sample 2 | Sample 3 | Sample 4 | Sample 5 | Sample 6 |
|---|---|---|---|---|---|---|
| indacaterol maleate (µg/mL) | 60 | 60 | 60 | 60 | 60 | 60 |
| glycopyrronium bromide (µg/mL) | 25 | 25 | 25 | 25 | 25 | 25 |
| pH | 2.5 | 3.0 | 3.5 | 4.0 | 4.5 | 5.0 |
| vehicle | water for injection | | | | | |

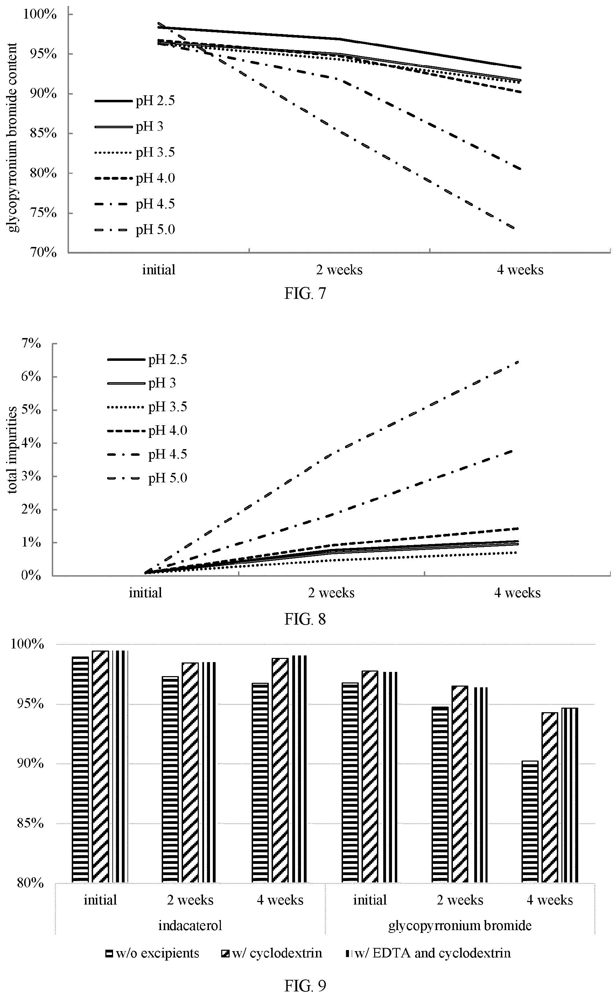

The drug solutions were placed in glass bottles and stability test was performed to determine the contents of indacaterol and glycopyrronium bromide and total impurities in the samples at the initial state and after 4 weeks at 60°C. Results are shown in Table 14 and FIG. 6 to 8. FIG. 6 shows the change in indacaterol content in the stability test of indacaterol maleate-glycopyrronium bromide compositions at different pH. FIG. 7 shows the change in glycopyrronium bromide content in the stability test of indacaterol maleate-glycopyrronium bromide composition with different excipients. FIG. 8 shows the change in total impurities in the stability test of indacaterol maleate-glycopyrronium bromide composition with different excipients.

**Table 14 Stability test results of the indacaterol maleate-glycopyrronium bromide compositions**

| | indacaterol content | | | glycopyrronium bromide content | | | total impurities | | |
|---|---|---|---|---|---|---|---|---|---|
| | 0 week | 2 weeks | 4 weeks | 0 week | 2 weeks | 4 weeks | 0 week | 2 weeks | 4 weeks |
| Sample 1 | 97.3% | 95.9% | 95.4% | 98.3% | 96.9% | 93.3% | 0.09% | 0.77% | 1.04% |
| Sample 2 | 99.5% | 97.7% | 97.5% | 96.4% | 95.0% | 91.7% | 0.09% | 0.69% | 0.95% |
| Sample 3 | 99.6% | 98.1% | 98.0% | 96.3% | 94.3% | 91.4% | 0.08% | 0.47% | 0.70% |
| Sample 4 | 98.9% | 97.3% | 96.7% | 96.7% | 94.7% | 90.2% | 0.09% | 0.91% | 1.42% |
| Sample 5 | 97.7% | 93.4% | 91.1% | 96.3% | 91.8% | 80.5% | 0.09% | 1.84% | 3.82% |
| Sample 6 | 98.8% | 92.3% | 88.3% | 98.8% | 85.3% | 72.6% | 0.09% | 3.67% | 6.44% |

The analysis of the changes in the stability period of the drug solutions at different pH shows that the stability of idacaterol and gycopyrronium bromide at pH 5 and pH 4.5 is poor. Indacaterol is stable at pH 2.5-4, the best at pH 3.5, while glycopyrronium bromide is stable at pH 2.5-3.5. Combining the contents of indacaterol and glycopyrronium bromide with the change in total impurities, it is determined that, in some embodiments, the optimal pH of the indacaterol-glycopyrronium bromide composition is 3.5.

### Example 8 Stability test of the indacaterol maleate-glycopyrronium bromide compositions with excipients

In addition, the effects of EDTA and cyclodextrin on the stability of the indacaterol maleate-glycopyrronium bromide compositions were investigated. The experimental formula is shown in Table 15. Citric acid and sodium citrate solutions were used to adjust the pH to target pH pH, and prepared drug solutions were filled into the glass bottle to seal.

**Table 15 Formula of the indacaterol maleate-glycopyrronium bromide compositions**

| | Sample 1 | Sample 2 | Sample 3 | Sample 4 | Sample 5 | Sample 6 |
|---|---|---|---|---|---|---|
| glycopyrronium bromide (µg/mL) | 25 | 25 | 25 | 25 | 25 | 25 |
| indacaterol maleate (µg/mL) | 60 | 60 | 60 | 60 | 60 | 60 |
| pH | 4.0 | | | 3.0 | | |
| EDTA(w/w) | 0 | 0 | 0.03% | 0 | 0.03% | 0.03% |
| cyclodextrin(w/w) | 0 | 0.50% | 0.50% | 0 | 0 | 0.50% |
| solvent | water for injection | | | | | |

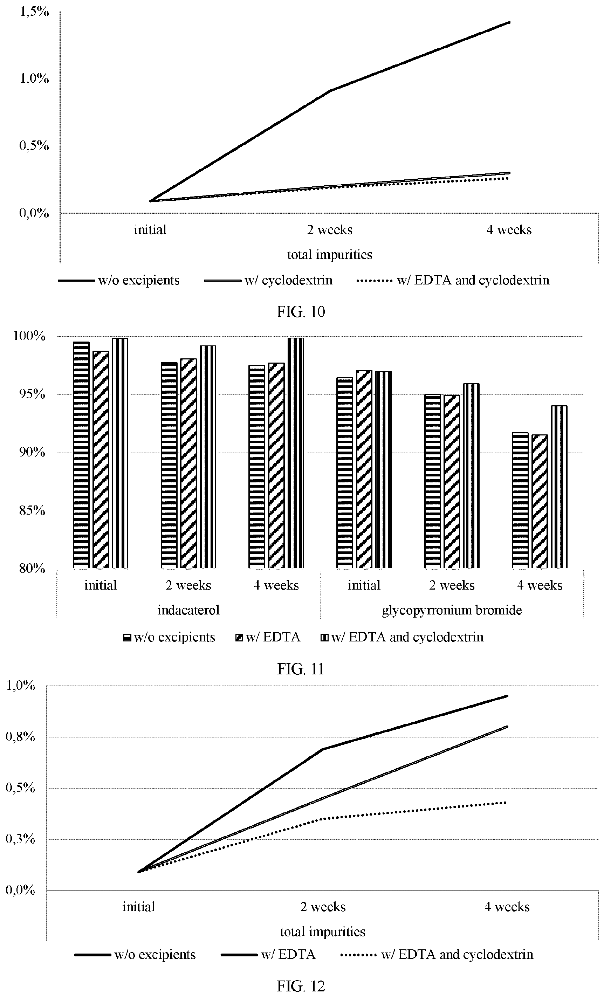

The drug solutions were placed in glass bottles and stability test was performed to determine the contents of indacaterol and glycopyrronium bromide and total impurities at initial and after 4 weeks at 60°C. The results are shown in Table 16 and FIGs. 9-12. FIG. 9 shows the changes in indacaterol content and glycopyrronium bromide content in the stability test of indacaterol maleate-glycopyrronium bromide composition at pH 4 with different excipients. FIG. 10 shows the changes in total impurities in the stability test of indacaterol maleate-glycopyrronium bromide composition at pH 4 with different excipients. FIG. 11 shows the changes in indacaterol content and glycopyrronium bromide content in the stability test of indacaterol maleate-glycopyrronium bromide composition at pH 3 with different excipients. FIG. 12 shows the changes in total impurities in the stability test of indacaterol maleate-glycopyrronium bromide composition at pH 3 with different excipients.

**Table 16 Stability test results of the indacaterol maleate-glycopyrronium bromide compositions**

| | indacaterol content | | | glycopyrronium bromide content | | | total impurities | | |
|---|---|---|---|---|---|---|---|---|---|
| | 0 week | 2weeks | 4weeks | 0 week | 2weeks | 4weeks | 0 week | 2weeks | 4weeks |
| Sample 1 | 98.9% | 97.3% | 96.7% | 96.7% | 94.7% | 90.2% | 0.09% | 0.91% | 1.42% |
| Sample 2 | 99.4% | 98.4% | 98.8% | 97.8% | 96.5% | 94.3% | 0.09% | 0.20% | 0.30% |
| Sample 3 | 99.4% | 98.5% | 99.0% | 97.6% | 96.4% | 94.6% | 0.09% | 0.19% | 0.26% |
| Sample 4 | 99.5% | 97.7% | 97.5% | 96.4% | 95.0% | 91.7% | 0.09% | 0.69% | 0.95% |
| Sample 5 | 98.7% | 98.0% | 97.7% | 97.0% | 94.9% | 91.5% | 0.09% | 0.45% | 0.80% |
| Sample 6 | 99.8% | 99.2% | 99.8% | 97.0% | 95.9% | 94.0% | 0.09% | 0.35% | 0.43% |

It can be seen that after the addition of cyclodextrin or both of cyclodextrin and EDTA to the composition at pH 4, the content of indacaterol and glycopyrronium bromide in the compositions decreased less, and the total impurities increased less, which shows that adding cyclodextrin, or cyclodextrin and EDTA, significantly improve the stability of the drug solution. In the composition at pH 3, the effects of adding EDTA and cyclodextrin were investigated. The stability trend indicates that the addition of EDTA can improve the stability of the composition, but the addition of EDTA and cyclodextrin at the same time can significantly improve the stability of the composition compared with the addition of EDTA alone.

### Example 9 Stability test of the indacaterol maleate-glycopyrronium bromide-sodium hyaluronate compositions with excipients

The solutions of indacaterol maleate, glycopyrronium bromide and sodium hyaluronate at different pH were prepared as follows. Eight 20 mM citric acid solution were prepared and the pH were adjusted to 2.5, 3.0, 3.5, 4.0, 4.5 and 5.0 with 1 M of sodium hydroxide. Added with glycopyrronium bromide and indacaterol maleate, stirred to dissolve. The effect of adding EDTA and cyclodextrin on the stability of the drug solution were investigated. The specific formula is shown in Table 17.

**Table 17 Formula of the indacaterol maleate-glycopyrronium bromide-sodium hyaluronate compositions**

| | Sample 1 | Sample 2 | Sample 3 | Sample 4 | Sample 5 | Sample 6 | Sample 7 | Sample 8 |
|---|---|---|---|---|---|---|---|---|
| glycopyrronium bromide (µg/mL) | 25 | 25 | 25 | 25 | 25 | 25 | 25 | 25 |
| indacaterol maleate (µg/mL) | 60 | 60 | 60 | 60 | 60 | 60 | 60 | 60 |
| pH | 4.0 | | | | 3.0 | | | |
| EDTA(w/w) | 0 | 0.03% | 0 | 0.03% | 0 | 0.03% | 0 | 0.03% |
| cyclodextrin(w/w) | 0 | 0 | 0.50% | 0.50% | 0 | 0 | 0.50% | 0.50% |
| sodium hyaluronate(w/w) | 0.3% | 0.3% | 0.3% | 0.3% | 0.3% | 0.3% | 0.3% | 0.3% |
| solvent | water for injection | | | | | | | |

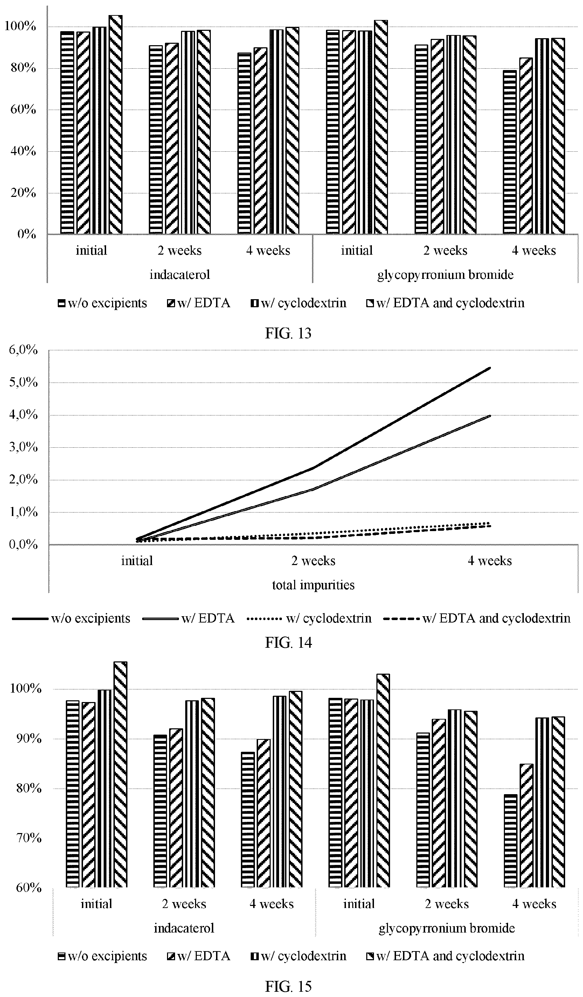

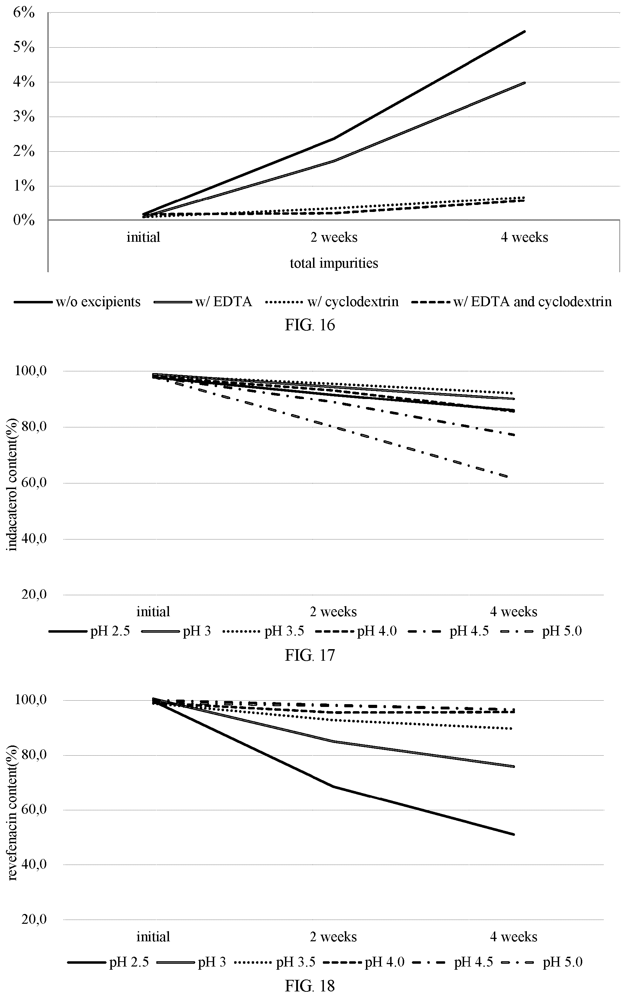

The drug solutions were filled in glass bottles and stability test was performed to determine the contents of indacaterol, glycopyrronium bromide, and total impurities at initial and after 4 weeks at 60°C. The results are shown in Table 18 and FIGs. 13-16. FIG. 13 shows the changes in indacaterol content and glycopyrronium bromide content in the stability test of indacaterol maleate-glycopyrronium bromide-sodium hyaluronate composition at pH 4 with different excipients. FIG. 14 shows the changes in total impurities in the stability test of indacaterol maleate-glycopyrronium bromide-sodium hyaluronate composition at pH 4 with different excipients. FIG. 15 shows the changes in indacaterol content and glycopyrronium bromide content in the stability test of indacaterol maleate-glycopyrronium bromide-sodium hyaluronate composition at pH 3 with different excipients. FIG. 16 shows the changes in total impurities in the stability test of indacaterol maleate-glycopyrronium bromide-sodium hyaluronate composition at pH 3 with different excipients.

**Table 18 Stability test results of the indacaterol maleate-glycopyrronium bromide-sodium hyaluronate compositions**

| | indacaterol content | | | glycopyrronium bromide content | | | total impurities | | |
|---|---|---|---|---|---|---|---|---|---|
| | 0 week | 2weeks | 4weeks | 0 week | 2weeks | 4weeks | 0 week | 2weeks | 4weeks |
| Sample 1 | 97.5% | 90.7% | 87.2% | 98.1% | 91.1% | 78.7% | 0.17% | 2.36% | 5.45% |
| Sample 2 | 97.3% | 92.0% | 89.8% | 97.9% | 93.9% | 84.9% | 0.09% | 1.71% | 3.97% |
| Sample 3 | 99.7% | 97.6% | 98.5% | 97.8% | 95.8% | 94.1% | 0.09% | 0.34% | 0.66% |
| Sample 4 | 105.4% | 98.1% | 99.5% | 103.0% | 95.5% | 94.4% | 0.17% | 0.20% | 0.57% |
| Sample 5 | 99.5% | 97.7% | 97.5% | 96.4% | 95.0% | 91.7% | 0.09% | 0.69% | 0.95% |
| Sample 6 | 98.7% | 98.0% | 97.7% | 97.0% | 94.9% | 91.5% | 0.09% | 0.45% | 0.80% |
| Sample 7 | 99.9% | 97.5% | 96.7% | 96.9% | 95.6% | 92.7% | 0.09% | 0.68% | 1.22% |
| Sample 8 | 99.8% | 99.2% | 99.8% | 97.0% | 95.9% | 94.0% | 0.09% | 0.35% | 0.43% |

It can be seen that the addition of excipients EDTA and cyclodextrin to the indacaterol maleate-glycopyrronium bromide-sodium hyaluronate compositions at pH 3.0 and 4.0 can improve the stability of the drug solution. Comparing the results of adding cyclodextrin and adding cyclodextrin and EDTA together, it can be seen that cyclodextrin has the better effect on improving the stability of the drug solution.

### Example 10 Stability test of the indacaterol maleate-revefenacin compositions at different pH

Citric acid/sodium citrate buffers at pH 2.5, 3.0, 3.5, 4.0, 5.5 were prepared at 20 mM concentrations. Indacaterol maleate was added according to the following prescription and ultrasoniced until completely dissolved. Sodium chloride and revefenacin were added and stirred until dissolved. The prepared drug solutions were filled into a glass bottle and sealed. The experimental formula is shown in Table 19.

**Table 19 Formula of the indacaterol maleate-revefenacin compositions**

| | Sample 1 | Sample 2 | Sample 3 | Sample 4 | Sample 5 | Sample 6 |
|---|---|---|---|---|---|---|
| indacaterol maleate (µg/mL) | 45 | 45 | 45 | 45 | 45 | 45 |
| revefenacin (µg/mL) | 85 | 85 | 85 | 85 | 85 | 85 |
| sodium chloride (%, w/w) | 0.85 | 0.85 | 0.85 | 0.85 | 0.85 | 0.85 |
| pH | 2.5 | 3.0 | 3.5 | 4.0 | 4.5 | 5.0 |
| Solvent | water for injection | | | | | |

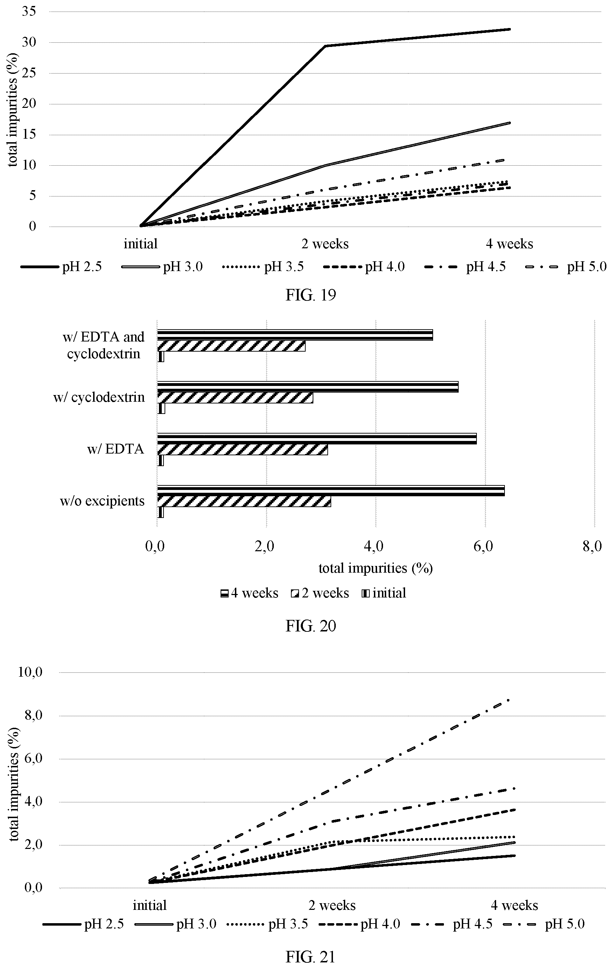

The samples were placed at a high temperature of 60 °C for 4 weeks for stability test to determine the changes in the contents of indacaterol, revefenacin and total impurities in the samples, as shown in Table 20 and FIGs. 17-19. FIG. 17 shows the change in indacaterol content in the stability test of indacaterol maleate-revefenacin compositions at different pH. FIG. 18 shows the change in indacaterol content in the stability test of indacaterol maleate-revefenacin compositions at different pH. FIG. 19 shows the change in total impurities in the stability test of indacaterol maleate-revefenacin compositions at different pH.

**Table 20 Stability test results of the indacaterol maleate-revefenacin compositions**

| | indacaterol content | | | revefenacin content | | | total impurities | | |
|---|---|---|---|---|---|---|---|---|---|
| | 0 week | 2 weeks | 4 weeks | 0 week | 2 weeks | 4 weeks | 0 week | 2 weeks | 4 weeks |
| Sample 1 | 97.8% | 91.5% | 85.9% | 99.7% | 68.5% | 50.9% | 0.18% | 29.4% | 32.13% |
| Sample 2 | 98.9% | 94.3% | 90.0% | 100.5% | 85.0% | 75.8% | 0.14% | 9.93% | 16.9% |
| Sample 3 | 98.4% | 95.5% | 92.1% | 98.8% | 92.8% | 89.6% | 0.12% | 4.13% | 7.39% |
| Sample 4 | 98.0% | 93.1% | 85.5% | 99.0% | 95.5% | 95.6% | 0.12% | 3.18% | 6.35% |
| Sample 5 | 97.9% | 88.9% | 77.1% | 100.0% | 98.1% | 96.6% | 0.13% | 3.67% | 7.00% |
| Sample 6 | 98.0% | 79.9% | 61.6% | 99.0% | 97.9% | 96.5% | 0.14% | 6.04% | 11.00% |

The results above show that the compound solution of indacaterol maleate-revefenacin is more stable at pH between 3.5 and 4.5 when placed at high temperature for 4 weeks than at pH 2.5 and 5.0.

### Example 11 Stability test of the indacaterol maleate-revefenacin compositions with excipients

In addition, the effects of adding EDTA (0.03% w/w) and cyclodextrin (0.05% w/w) to indacaterol maleate-revefenacin compositions at pH 4 in Example 10 on stability of the solution were investigated. The experimental formula is shown in Table 21. Citric acid and sodium citrate solution were used to adjust the pH to target pH, and the prepared drug solutions were filled into the glass bottle to seal.

**Table 21 Formula of the indacaterol maleate-revefenacin compositions with excipients**

| | Sample 1 | Sample 2 | Sample 3 | Sample 4 |
|---|---|---|---|---|
| indacaterol maleate (µg/mL) | 45 | 45 | 45 | 45 |
| revefenacin (µg/mL) | 85 | 85 | 85 | 85 |
| pH | 4.0 | | | |
| EDTA(w/w) | 0 | 0.03% | 0 | 0.03% |
| cyclodextrin(w/w) | 0 | 0 | 0.05% | 0.05% |
| solvent | water for injection | | | |

The samples were placed at a high temperature of 60 °C for 4 weeks, and the stability test was carried out to determine the change in the total impurities in the sample, as shown in Table 22 and FIG. 20. FIG. 20 shows the change in the total impurities in the stability test of Indacaterol maleate-revefenacin compositions at pH 4 with different excipients.

**Table 22 Results of the change of total impurities in the indacaterol maleate-revefenacin compositions with excipients**

| | initial | 2 weeks | 4 weeks |
|---|---|---|---|
| Sample 1 | 0.12% | 3.18% | 6.35% |
| Sample 2 | 0.12% | 3.12% | 5.84% |
| Sample 3 | 0.15% | 2.85% | 5.51% |
| Sample 4 | 0.13% | 2.71% | 5.04% |

The results show that the addition of cyclodextrin may improve the stability of the drug solution more significantly. If both cyclodextrin and EDTA are added to the drug solution, the stability of the drug solution may be significantly improved.

### Example 12 Stability test of the indacaterol maleate-tiotropium bromide compositions at different pH

Citric acid/sodium citrate buffers at pH 2.5, 3.0, 3.5, 4.0, 5.5 were prepared at 20 mM concentrations. Indacaterol maleate was added according to the following prescription and ultrasoniced until completely dissolved. Sodium chloride and totropium bromide were added and stirred until dissolved. The prepared drug solutions were filled into a glass bottle and sealed. The experimental formula is shown in Table 23.

**Table 23 Formula of the indacaterol maleate-tiotropium bromide compositions**

| | Sample 1 | Sample 2 | Sample 3 | Sample 4 | Sample 5 | Sample 6 |
|---|---|---|---|---|---|---|
| indacaterol maleate (µg/mL) | 45 | 45 | 45 | 45 | 45 | 45 |
| tiotropium bromide (µg/mL) | 30 | 30 | 30 | 30 | 30 | 30 |
| sodium chloride (%, w/w) | 0.85 | 0.85 | 0.85 | 0.85 | 0.85 | 0.85 |
| pH | 2.5 | 3.0 | 3.5 | 4.0 | 4.5 | 5.0 |
| solvent | water for injection | | | | | |

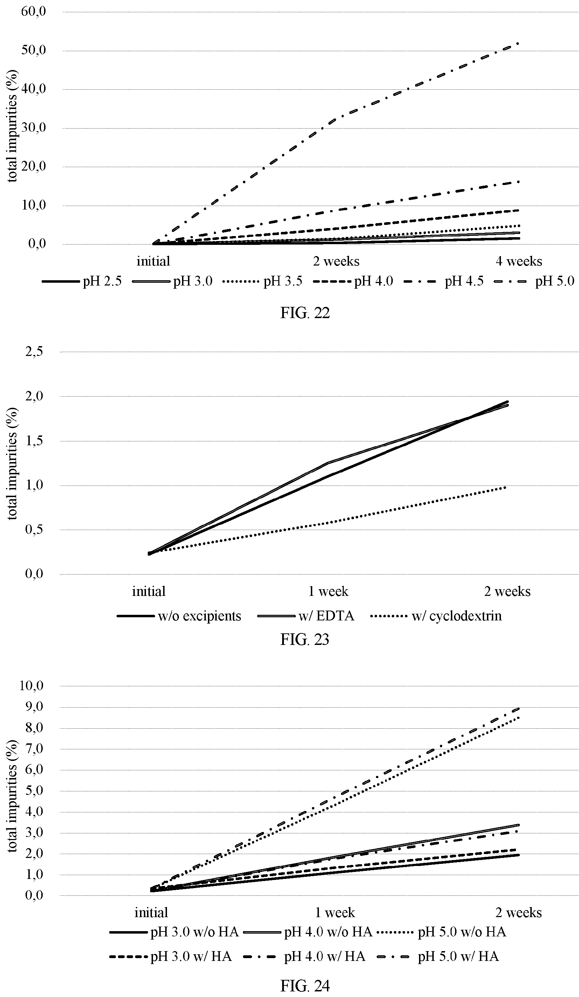

The samples were placed at a high temperature of 60 °C for 4 weeks for stability test, and the contents of indacaterol and tiotropium bromide and the change in total impurities in the samples were determined, as shown in Table 24 and FIGs. 21-22. FIG. 21 shows the change in total indacaterol impurities in the stability test of indacaterol maleate-tiotropium bromide compositions at different pH. FIG. 22 shows the change in total tiotropium bromide impurities in the stability test of indacaterol maleate-tiotropium bromide compositions at different pH.

**Table 24 Stability test results of the indacaterol maleate-tiotropium bromide compositions**

| | indacaterol content | | | tiotropium bromide content | | | indacaterol total impurities | | | tiotropium bromide total impurities | | |
|---|---|---|---|---|---|---|---|---|---|---|---|---|
| | initial | 2 weeks | 4 weeks | initial | 2 weeks | 4 weeks | initial | 2 weeks | 4 weeks | initial | 2 weeks | 4 weeks |
| Sample 1 | 97.1% | 97.7% | 97.6% | 99.2% | 99.7% | 97.3% | 0.25% | 0.88% | 1.50% | 0.00% | 0.30% | 1.45% |
| Sample 2 | 95.5% | 97.7% | 95.0% | 100.1% | 98.9% | 96.6% | 0.24% | 0.88% | 2.11% | 0.00% | 1.13% | 2.97% |
| Sample 3 | 95.8% | 95.4% | 91.6% | 99.3% | 97.9% | 94.1% | 0.26% | 2.14% | 2.37% | 0.00% | 1.37% | 4.74% |
| Sample 4 | 97.2% | 95.7% | 93.9% | 100.0% | 96.0% | 90.8% | 0.24% | 1.98% | 3.63% | 0.03% | 4.04% | 8.74% |
| Sample 5 | 96.2% | 94.5% | 93.4% | 99.2% | 91.3% | 82.7% | 0.26% | 3.09% | 4.62% | 0.00% | 8.72% | 16.14% |
| Sample 6 | 93.5% | 91.9% | 86.7% | 100.5% | 67.6% | 48.0% | 0.35% | 4.59% | 8.86% | 0.03% | 32.38% | 51.95% |

The results show that the content of indacaterol decrease by less than 5% when the pH of the sample is 2.5-4.5, and the content of tiotropium bromide decrease by less than 5% when the pH of the sample is 2.5-3.5. At the same time, combined with the analysis of the total impurities of indacaterol and tiotropium bromide, it is preferred to select the appropriate pH of the indacaterol maleate-tiotropium bromide composition between 2.5 and 3.5.

### Example 13 Stability test of the indacaterol maleate-tiotropium bromide compositions with excipients

In addition, the effects of adding excipients such as EDTA (0.03% w/w) and cyclodextrin (0.05% w/w) to indacaterol maleate-tiotropium bromide composition at pH 3 in Example 12 on stability were investigated. The experimental formula is shown in Table 25. Citric acid and sodium citrate solution were used to adjust the pH to target pH, and the prepared drug solutions were filled into the glass bottle to seal.

**Table 25 Formula of the indacaterol maleate-tiotropium bromide compositions with excipients**

| | Sample 1 | Sample 2 | Sample 3 |
|---|---|---|---|
| indacaterol maleate (µg/mL) | 45 | 45 | 45 |
| tiotropium bromide (µg/mL) | 30 | 30 | 30 |
| pH | 3.0 | | |
| EDTA(w/w) | 0 | 0.03% | 0 |
| cyclodextrin(w/w) | 0 | 0 | 0.05% |
| solvent | water for injection | | |

The samples were placed at a high temperature of 60 °C for 4 weeks to conduct stability test and test the change in total impurities in the sample, as shown in Table 26 and FIG. 23. FIG. 23 shows the change in total impurities in the stability test of indacaterol maleate-tiotropium bromide compositions at pH 3 with different excipients.

**Table 26 Results of the change in total impurities in the indacaterol maleate-tiotropium bromide compositions with excipients**

| | initial | 2weeks | 4 weeks |
|---|---|---|---|
| Sample 1 | 0.22% | 1.10% | 1.94% |
| Sample 2 | 0.23% | 1.25% | 1.90% |
| Sample 3 | 0.24% | 0.58% | 0.98% |

According to the results of total impurity content changes in Table 26 and FIG. 23, it can be seen that adding cyclodextrin can significantly improve the stability of the drug solution compared with adding no excipients or adding EDTA.

### Example 14 Stability test of the indacaterol maleate-tiotropium bromide compositions with excipients

In addition, the effects of adding sodium hyaluronate to indacaterol maleate-tiotropium bromide compositions at pH 3, 4, and 5 in Example 12 were investigated. The experimental formula is shown in Table 27. Citric acid and sodium citrate solution were used to adjust the pH to target pH. The prepared drug solutions were filled into a glass bottle and sealed.

**Table 27 Formulation of indacaterol maleate-tiotropium bromide compositions with excipients**

| | Sample 1 | Sample 2 | Sample 3 | Sample 4 | Sample 5 | Sample 6 |
|---|---|---|---|---|---|---|
| indacaterol maleate (µg/mL) | 45 | 45 | 45 | 45 | 45 | 45 |
| tiotropium bromide (µg/mL) | 30 | 30 | 30 | 30 | 30 | 30 |
| sodium chloride (w/w) | 0.85% | 0.85% | 0.85% | 0.85% | 0.85% | 0.85% |
| sodium hyaluronate (w/w) | 0.00 | | | 0.30% | | |
| pH | 3.0 | 4.0 | 5.0 | 3.0 | 4.0 | 5.0 |
| Solvent | water for injection | | | | | |

The samples were placed at a high temperature of 60 °C for 4 weeks, and stability test was conducted to determine the change in total impurities in the sample, as shown in Table 28 and FIG. 24. FIG. 24 shows the change in total impurities during stability test of indacaterol maleate-tiotropium bromide compositions at pH 3, 4, or 5 with or without sodium hyaluronate. The terms "w/o HA" and "w/ HA" in FIG. 24 mean "without sodium hyaluronate added" and "with sodium hyaluronate added" respectively.

**Table 28 Effect of sodium hyaluronate added to indacaterol maleate-tiotropium bromide composition on the change in total impurities**

| | initial | 1 week | 2 weeks |
|---|---|---|---|
| Sample 1 | 0.22% | 1.10% | 1.94% |
| Sample 2 | 0.22% | 1.84% | 3.37% |
| Sample 3 | 0.32% | 4.34% | 8.49% |
| Sample 4 | 0.34% | 1.33% | 2.20% |
| Sample 5 | 0.22% | 1.77% | 3.08% |
| Sample 6 | 0.33% | 4.70% | 8.92% |

According to the results of total impurities changes in Table 28 and FIG. 24, there is no significant change in the stability of the drug solution after sodium hyaluronate added, and the trend of stability change is consistent with that before sodium hyaluronate added.

### Example 15 Preparation of long-acting β₂ agonist-long-acting muscarinic antagonist-hyaluronic acid pharmaceutical composition

### Preparation of olodaterol hydrochloride-revefenacin-hyaluronic acid spray

The prescribed amounts of olodaterol hydrochloride, revefenacin, high molecular weight hyaluronic acid, and sodium calcium edetate were weighed accurately and added with the prescribed amounts of ethanol and stirred well. The appropriate amount of water for injection and benzalkonium chloride were added, and the pH was adjusted with citric acid/sodium citrate buffer to 4.5 to 5.5, then filled to a sprayer. The specific composition of the prepared spray is shown in Table 29.

**Table 29 Composition of olodaterol hydrochloride-revefenacin-hyaluronic acid spray**

| components | mass/spray |
|---|---|
| olodaterol hydrochloride | 2.7 µg/spray |
| revefenacin | 40µg |
| high molecular weight hyaluronic acid | appropriate amount |
| sodium calcium edetate | 0.03% of the total formula |
| ethanol | 1% |
| tween 80 | 1% |
| benzalkonium chloride | appropriate amount |
| citric acid | amount to adjust pH to 4.5-5.5 |
| sodium citrate | |
| water for injection | fill to volume |

### Preparation of olodaterol hydrochloride-glycopyrronium bromide-hyaluronic acid aerosol

The prescribed amounts of ethyl oleate and Tween 80 were weighed and mixed well. The prescribed amounts of olodaterol hydrochloride, glycopyrronium bromide and high molecular weight hyaluronic acid were added and mixed well. The prepared drug solution was transferred to a mixing container with a hydrofluoroalkane (HFA) ejector. The specific composition of the prepared aerosol is shown in Table 30.

**Table 30 Composition of olodaterol hydrochloride-glycopyrronium bromide-hyaluronic acid aerosol**

| component | mass/spray |
|---|---|
| olodaterol hydrochloride | 5 µg |
| glycopyrronium bromide | 50 µg |
| high molecular weight hyaluronic acid | 50 µg |
| ethyl oleate | 100 µg |
| tween 80 | 100 µg |
| 1,1,1,2-tetrafluoroethane (HFA-134(a)) or 1,1,1,2,3,3,3-heptafluoropropane (HFA-227) | *q.s.* |

### Preparation of olodaterol hydrochloride-glycopyrronium bromide-hyaluronic acid aerosol

The prescribed amounts of glycerin and ethanol were weighed and mixed well, accurately added with the prescribed amounts of olodaterol hydrochloride with glycopyrronium bromide and high molecular weight hyaluronic acid, and mixed well. The pH was adjusted to 4.5-5.5 with dilute hydrochloric acid and transferred to a mixing container with hydrofluoroalkane (HFA) added. The specific composition of the prepared inhalation aerosol is shown in Table 31.

**Table 31 Composition of olodaterol hydrochloride-glycopyrronium bromide-hyaluronic acid aerosol**

| Component | mass/spray |
|---|---|
| olodaterol hydrochloride | 5 µg |
| glycopyrronium bromide | 50 µg |
| high molecular weight hyaluronic acid | appropriate amount |
| ethanol | 15-20% of the total formula |
| glycerinum (0.08 N) | 1% of the total formula |
| dilute hydrochloric acid (0.08%) | amount to adjust pH to 4.5-5.5 |
| 1,1,1,2-tetrafluoroethane (HFA-134(a)) or 1,1,1,2,3,3,3-heptafluoropropane (HFA-227) | *q.s.* |

### Preparation of olodaterol hydrochloride-revefenacin-hyaluronic acid spray

The prescribed amounts of disodium edetate was weighed and mixed well, accurately added with the prescribed amounts of olodaterol hydrochloride, revefenacin and high molecular weight hyaluronic acid, and mixed well. pH was adjusted to 4.5 to 5.5 with citric acid and filled to prepared for an inhalation spray. The specific composition of the prepared inhalation spray is shown in Table 32.

**Table 32 Composition of olodaterol hydrochloride-revefenacin-hyaluronic acid spray**

| component | mass/spray |
|---|---|
| olodaterol hydrochloride | 5 µg |
| revefenacin | 80 µg |
| high molecular weight hyaluronic acid | appropriate amount |
| disodium edetate | 0.25 µg |
| citric acid | amount to adjust pH to 4.5-5.5 |
| water for injection | *q.s.* |

### Preparation of olodaterol hydrochloride-revefenacin-hyaluronic acid aerosol

The prescribed amounts of propylene glycol and ethanol were weighed and mixed well, accurately added with the prescribed amounts of olodaterol hydrochloride, revefenacin and high molecular weight hyaluronic acid, and mixed well. The prepared drug solution was transferred to a mixing container with HFA, mixed, recycled and filled into a special container. The specific composition of the prepared aerosol is shown in Table 33.

**Table 33 Composition of olodaterol hydrochloride-revefenacin-hyaluronic acid aerosol**

| component | mass/spray |
|---|---|
| olodaterol hydrochloride | 5 µg |
| revefenacin | 40 µg |
| high molecular weight hyaluronic acid | appropriate amount |
| propylene glycol | 1% |
| ethanol | 3% |
| 1,1,1,2-tetrafluoroethane (HFA-134(a)) | *q.s.* |

### Preparation of vilanterol-glycopyrronium bromide-hyaluronic acid aerosol

The prescribed amounts of ethyl oleate and tween 80 were weighed and mixed well. The prescribed amounts of vilanterol, glycopyrronium bromide and high molecular weight hyaluronic acid were precisely added and mixed well. The prepared solution was transferred to a mixing container with HFA. The specific formula is shown in Table 34.

**Table 34 Composition of glycopyrronium bromide-vilanterol-hyaluronic acid aerosol**

| component | mass/spray |
|---|---|
| vilanterol | 5 µg |
| glycopyrronium bromide | 50 µg |
| high molecular weight hyaluronic acid | appropriate amount |
| ethyl oleate | 100 g |
| Tween 80 | 100 g |
| 1,1,1,2-tetrafluoroethane (HFA-134(a)) or 1, 1, 1,2,3,3,3-heptafluoropropane(HFA-227) | *q.s.* |

### Preparation of vilanterol-glycopyrronium bromide-hyaluronic acid inhalation solution

The prescribed amounts of sodium chloride and EDTA were weighed and dissolved with water for injection, then mixed well. The prescribed amounts of vilanterol, glycopyrronium bromide and high molecular weight hyaluronic acid were added and mixed well. The prepared drug solution was filled into a low-density polyethylene vial and sealed with aluminum foil. See Table 35 for specific formulations.

**Table 35 Composition of glycopyrronium bromide-vilanterol-hyaluronic acid inhalation solution**

| component | mass / inhalation solution |
|---|---|
| glycopyrronium bromide | 0.086% |
| vilanterol | 0.006% |
| high molecular weight hyaluronic acid | 0.03% |
| citric acid | amount to adjust pH to 3-5 |
| sodium citrate | |
| sodium chloride | 0.85% |
| EDTA | 0.001% |
| water for injection | 99.2567% |

### Preparation of olodaterol hydrochloride-glycopyrronium bromide-hyaluronic acid inhalation solution

The prescribed amounts of sodium chloride and EDTA were weighed and dissolved with water for injection, and mixed well. the prescribed amounts of olodaterol, glycopyrronium bromide and high molecular weight hyaluronic acid were added and mixed well. The prepared drug solutions were filled into a low-density polyethylene vial and sealed with aluminum foil. See Table 36 for specific formulations.

**Table 36 Composition of glycopyrronium bromide-olodaterol-hyaluronic acid inhalation solution**

| component | mass / inhalation solution |
|---|---|
| glycopyrronium bromide | 0.086% |
| olodaterol hydrochloride | 0.006% |
| high molecular weight hyaluronic acid | 0.30% |
| citric acid | amount to adjust pH to 3-5 |
| sodium citrate | |
| sodium chloride | 0.85% |
| EDTA | 0.001% |
| water for injection | 99.2567% |

The above embodiments are only the preferred embodiments of the present application and cannot be used to limit the scope of protection in the present application. Any non-substantive changes and substitutions made on the basis of the present application by a person skilled in the field shall fall within the scope of the protection claimed in the present application.

## Claims

1. A pharmaceutical composition for inhalation, comprising:
a long-acting β₂ agonist or pharmaceutically acceptable salt thereof; and
a long-acting muscarinic antagonist or pharmaceutically acceptable salt thereof,
wherein
the long-acting β₂ agonist comprises olodaterol; or
the long-acting β₂ agonist comprises arformoterol, and the pH value of the composition is 3.5-5.5; or
the long-acting β₂ agonist comprises indacaterol, and the pH value of the composition is 2.5-4.5; or
the long-acting muscarinic antagonist comprises revefenacin, and the pH value of the composition is 3.5-5.5; or
the long-acting muscarinic antagonist comprises glycopyrronium bromide, and the pH value of the composition is 2.5 to 4.5; or
the long-acting muscarinic antagonist comprises tiotropium bromide, and the pH value of the composition is 2.0 to 4.0.

2. The composition according to claim 1, wherein the long-acting β₂ agonist is at least one selected from a group consisting of indacaterol, formoterol, arformoterol, vilanterol, carmoterol, and olodaterol.

3. The composition according to claim 1 or claim 2, wherein the long-acting muscarinic agonist is at least one selected from a group consisting of glycopyrronium bromide, umeclidinium bromide, tiotropium bromide, aclidinium bromide, and revefenacin.

4. The composition according to any one of claims 1 to 3, wherein the long-acting β₂ agonist or pharmaceutically acceptable salt thereof has an amount of 1-100 µg/mL, optionally 5-100 µg/mL.

5. The composition according to any one of claims 1 to 4, wherein the long-acting muscarinic antagonist or pharmaceutically acceptable salt thereof has an amount of 1-100 µg/mL , optionally 5-100 µg/mL.

6. The composition according to any one of claims 1 to 5, wherein a weight ratio of the long-acting β₂ agonist or pharmaceutically acceptable salt thereof to the long-acting muscarinic antagonist or a pharmaceutically acceptable salt thereof is 100 : 1-1 : 100, optionally 1: 80-80 : 1, optionally 1: 60-60 : 1, optionally 1: 50-50 : 1, optionally 1: 40-40 : 1, optionally 1: 30-30 : 1, optionally 1: 20-20 : 1, optionally 1: 15-15 : 1.

7. The composition according to any one of claims 1 to 6, wherein the long-acting β₂ agonist comprises olodaterol, and the long-acting muscarinic antagonist comprises revefenacin;
optionally, the composition has a pH value of 4.0-5.5;
optionally, the composition further comprises EDTA.

8. The composition according to any one of claims 1 to 6, wherein the long-acting β₂ agonist comprises arformoterol, the long-acting muscarinic antagonists comprises revefenacin, and the composition has a pH value of 4.0-5.5;
optionally, the composition has a pH value of 4.5-5.5;
optionally, the composition has a pH value of 4.0-5.0;
optionally, the composition further comprises EDTA;
optionally, the composition further comprises buffer;
optionally, the buffer is citric acid/citrate buffer;
optionally, the buffer is citric acid/sodium citrate buffer;
optionally, the buffer concentration ranges from 0.1 mM to 20 mM;
optionally, the composition further comprises sodium chloride;
optionally, in the composition, the sodium chloride has a weight percentage of 0.01%-1.00%

9. The composition according to any one of claims 1 to 6, wherein the long-acting β₂ agonist comprises indacaterol, the long-acting muscarinic antagonist comprises glycopyrronium bromide, and the composition has a pH value of 2.5-4.0;
optionally, the composition has a pH value of 3.0-4.0;
optionally, the composition has a pH value of 2.5-3.5;
optionally, the composition has a pH value of 3.0-3.5;
optionally, the composition further comprises EDTA and/or cyclodextrin;
optionally, the composition further comprises cyclodextrin;
optionally, the composition further comprises EDTA and cyclodextrin;
optionally, the composition further comprises sodium hyaluronate;
optionally, the composition further comprises sodium hyaluronate and at least one selected from a group consisting of EDTA and cyclodextrin;
optionally, the composition further comprises sodium hyaluronate and cyclodextrin.

10. The composition according to any one of claims 1 to 6, wherein the long-acting β₂ agonist comprises indacaterol, the long-acting muscarinic antagonist comprises revefenacin, and the composition has a pH value of 3.0-4.5;
optionally, the composition has a pH value of 3.5-4.5;
optionally, the composition has a pH value of 3.5-4.0;
optionally, the composition has a pH value of 4.0-4.5;
optionally, the composition further comprises EDTA and/or cyclodextrin;
optionally, the composition further comprises cyclodextrin;
optionally, the composition further comprises EDTA and cyclodextrin.

11. The composition according to any one of claims 1 to 6, wherein the long-acting β₂ agonist comprises indacaterol, the long-acting muscarinic antagonist comprises tiotropium bromide, and the composition has a pH value of 2.0-4.0;
optionally, the composition has a pH value of 2.5-4.0;
optionally, the composition has a pH value of 2.5-3.5;
optionally, the composition has a pH value of 2.5-3.0;
optionally, the composition further comprises cyclodextrin.

12. The composition according to any one of claims 1 to 11, further comprising hyaluronic acid or pharmaceutically acceptable salt thereof;
optionally, the hyaluronic acid is high molecular weight hyaluronic acid;
optionally, the hyaluronic acid has a weight percentage of 0.01%-0.50%.

13. The composition according to any one of claims 1 to 12, further comprising sodium chloride, EDTA or pharmaceutically acceptable salt thereof, and/or cyclodextrin or derivatives thereof or pharmaceutically acceptable salt thereof;
optionally, the cyclodextrin comprises α-cyclodextrin, β-cyclodextrin or γ-cyclodextrin;
optionally, the cyclodextrin comprises hydroxypropyl-β-cyclodextrin or sulfobutyl-β-cyclodextrin;
optionally, the sodium chloride has a weight percentage of is 0.01%-1.00%;
optionally, the EDTA or pharmaceutically acceptable salt thereof has a weight percentage of 0.001%-0.100%;
optionally, the cyclodextrin has a weight percentage of 0.01%-10%, optionally 0.01%-1.00%.

14. The composition according to any one of claims 1 to 13 for use in preventing or treating a respiratory disease, in particular as the pharmaceutical composition for inhalation in preventing or treating a respiratory disease.

15. The composition for use according to claim 14, wherein the respiratory disease comprises asthma or chronic obstructive pulmonary disease.
